(19) Europäisches Patentamt / European Patent Office / Office européen des brevets

(11) EP 2 753 300 B1

(12) # FASCICULE DE BREVET EUROPEEN

(45) Date de publication et mention de la délivrance du brevet:
**12.12.2018 Bulletin 2018/50**

(21) Numéro de dépôt: **12767032.1**

(22) Date de dépôt: **06.09.2012**

(51) Int Cl.:
***A61K 8/60*** *(2006.01)* ***A61K 8/73*** *(2006.01)*
***A61Q 19/08*** *(2006.01)*

(86) Numéro de dépôt international:
**PCT/FR2012/051991**

(87) Numéro de publication internationale:
**WO 2013/034855 (14.03.2013 Gazette 2013/11)**

(54) **ASSOCIATION DE CARRAGHÉNANE ET DE C-GLYCOSIDE ET LEURS UTILISATIONS**

KOMBINATION AUS CARRAGEENAN UND C-GLYCOSID UND VERWENDUNGEN DAVON

COMBINATION OF CARRAGEENAN AND C-GLYCOSIDE AND USES THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **06.09.2011 FR 1102709**
**23.09.2011 US 201161538269 P**

(43) Date de publication de la demande:
**16.07.2014 Bulletin 2014/29**

(73) Titulaire: **L'Oréal**
**75008 Paris (FR)**

(72) Inventeurs:
- **POTTER, Anne**
**F-92200 Neuilly-sur-Seine (FR)**
- **BALTENNECK, Carine**
**F-77500 Chelles (FR)**
- **SCHULTZE, Xavier**
**F-93320 les Pavillons sous Bois (FR)**
- **PRUDHOMME, Estelle**
**F-75018 Paris (FR)**

(74) Mandataire: **Brohmi, Karim**
**L'Oréal**
**Service DIPI**
**9 Rue Pierre Dreyfus**
**92110 Clichy (FR)**

(56) Documents cités:
**EP-A1- 2 204 162**
**WO-A1-2008/110672**
**WO-A2-2007/128939**
**FR-A1- 2 903 003**
**JP-A- 5 051 311**
**US-A- 4 543 250**
**US-B1- 6 572 868**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne des compositions, notamment cosmétiques, et des associations montrant un effet amélioré sur la maturation de l'enveloppe cornée. Elle concerne aussi l'utilisation des ces associations et compositions, ainsi qu'un procédé de traitement cosmétique, notamment pour favoriser le maintien d'une peau ayant une fonction barrière de bonne qualité et des propriétés biomécaniques optimales.

La peau est un tissu dont les cellules sont jointives, et solidaires les unes des autres. Le tissu cutané forme un revêtement externe comprenant des glandes sébacées ou sudoripares, et les follicules pileux. La peau, et notamment le cuir chevelu, sont des épithéliums à renouvellement continuel. Le renouvellement, ou desquamation, est un processus coordonné et finement régulé aboutissant à l'élimination des cellules superficielles, de façon insensible et non visible.
La peau humaine est constituée de deux compartiments à savoir un compartiment superficiel, l'épiderme, et un compartiment profond, le derme.
L'épiderme est conventionnellement divisé en une couche basale de kératinocytes constituant la couche germinative de l'épiderme, une couche dite épineuse constituée de plusieurs couches de cellules polyédriques disposées sur les couches germinatives, une à trois couches dites granuleuses constituées de cellules aplaties contenant des inclusions cytoplasmiques distinctes, les grains de kératohyaline et enfin, un ensemble de couches supérieures appelées couches cornées (ou *stratum corneum*), constituée de kératinocytes au stade terminal de leur différenciation appelés cornéocytes. Les cornéocytes sont des cellules anucléées principalement constituées d'une matière fibreuse contenant des cytokératines, entourée d'une enveloppe cornée. Il y a en permanence production de nouveaux kératinocytes pour compenser la perte en continu de cellules épidermiques au niveau de la couche cornée selon un mécanisme dénommé desquamation.
Toutefois, un déséquilibre entre la production des cellules au niveau de la couche basale et le taux de desquamation peut notamment conduire à des formations d'écailles à la surface de la peau. De même, un déficit de différentiation terminale des cellules du *stratum corneum,* pour diverses raisons, peut conduire à la formation d'amas de cellules de grandes tailles, épais, visibles à l'oeil nu, et dénommés « squames », ou dans d'autres situations, à un amincissement du *stratum corneum.* Les troubles de cette différentiation terminale peuvent aboutir à une fragilité, voire un défaut des propriétés barrières de l'épiderme, à une déshydratation chronique du stratum corneum, une perte d'élasticité mécanique, des tiraillements, ainsi qu'à un manque d'éclat et de transparence de la peau.
A titre d'exemple de facteurs favorisant cette altération de la qualité de surface de la peau, on peut mentionner le stress, la période hivernale, un excès de sébum, un défaut d'hydratation. Ces désordres apparaissent aussi dans le cas des peaux sèches de sujets âgés.
Ainsi une altération de la barrière cutanée peut se produire en présence d'agressions externes de type agents irritants (détergents, acides, bases, oxydants, réducteurs, solvants concentrés, gaz ou fumées toxiques), sollicitations mécaniques (frottements, chocs, abrasion, arrachement de la surface, projection de poussières, de particules, rasage ou épilation), déséquilibres thermiques ou climatiques (froid, sécheresse, radiations), xénobiotiques (micro-organismes indésirable, allergènes) ou d'agressions internes de type stress psychologique.

L'une des étapes critiques dans le processus de différentiation terminale du stratum corneum est la réticulation des précurseurs protéiques l'enveloppe cornée (EC). Ce phénomène joue un rôle essentiel dans le développement et le maintien de la cohésion cutanée, les propriétés physiques de la peau comme la fonction barrière, et est une étape cruciale dans le processus de différentiation terminale.
L'enveloppe cornée est un composant essentiel des cornéocytes. Chez les sujets en bonne santé, une altération de la fonction barrière, en particulier du visage, peut être associée à la présence d'enveloppe cornée immature et fragile dans les couches superficielles du stratum corneum (Hirao T et al. IFSCC Mag 2002; 6(2):103-109).
La maturation des EC depuis les couches profondes jusqu'aux couches superficielles du stratum corneum peut être caractérisée par des paramètres morphologiques et biophysiques ou mécaniques.

Les actifs hydratants classiquement utilisés, comme les humectants, les polymères hydratants, les corps gras comme la vaseline modifient de façon transitoire les propriétés superficielles de la peau. Ces actifs peuvent entraîner un assouplissement mécanique du stratum corneum, une augmentation de son état d'hydratation, une amélioration du microrelief de la peau par formation d'un film en surface de la peau. Généralement, ces effets ne sont pas rémanents dans le temps et ne durent que quelques heures. De plus, après nettoyage de la peau, ces actifs sont éliminés et l'effet d'assouplissement mécanique de la peau, l'amélioration de la texture de la peau ou de ses propriétés optiques disparaissent.

Par ailleurs, l'utilisation de filmogènes sur la peau, en particulier l'utilisation de polysaccharides humectants et hydrophiles de masse molaire élevée, conduit souvent à un effet "tenseur" de la peau, une augmentation du module élastique de la peau et cette rigidification de surface entraîne un inconfort de la peau.

Ces polymères, souvent chargés, peuvent également conduire à un épaississement trop important de la formule et leur utilisation limite les capacités formulatoires et la recherche de textures nouvelles. Il est alors difficile de les utiliser à une concentration élevée dans la formule, ce qui va limiter leur impact biologique.

Enfin, les peaux sèches, qui manquent d'éclat, sont souvent traitées par des actifs hydratants, qui ont un effet sur la différenciation et la maturation du stratum corneum. Ces actifs ont rarement un effet anti-âge, prolifératif, stimulant la régénération épidermique et conduisent rarement à une augmentation de l'épaisseur de l'épiderme vivant (action anti-âge). Ces deux mécanismes sont généralement antagonistes.

Il existe donc un besoin d'actifs améliorant l'état de surface de la peau, en particulier des peaux sèches ou âgées, en évitant les tiraillements et les sensations d'inconfort de l'utilisateur; il existe un besoin pour d'actifs, qui améliorent en outre les propriétés de texture et d'éclat du teint.

Le maintien ou le rétablissement d'une enveloppe cornée présentant une maturation correcte est essentiel pour préserver une fonction barrière de bonne qualité assurant une protection contre les agressions externes et une hydratation durable de la peau, en particulier de l'épiderme.

De manière inattendue, il a été trouvé que ces buts et d'autres peuvent être atteints avec une association de carraghénanes et de C-glycoside, qui présente des propriétés améliorées pour favoriser la maturation des enveloppes cornées des cornéocytes; cette induction de la différentiation permettant une amélioration durable de la qualité de surface de la peau et/ou de ses propriétés biophysiques. Une telle association trouve donc de nombreuses applications, en particulier dans le domaine cosmétique.

La demande WO02-051828 décrit de nouveaux dérivés C-glycosides et leur utilisation pour favoriser la synthèse des GAG par les fibroblastes de la peau, et ainsi lutter contre les signes du vieillissement et la sécheresse cutanée.

La demande FR2903003 décrit l'utilisation de dérivés C-glycosides pour renforcer la fonction barrière.

Les carraghénanes sont des polysaccharides extraits d'algues, utilisés dans le domaine alimentaire comme gélifiants.
FR 2917971 décrit l'utilisation d'oligosaccharides obtenus par hydrolyse de carraghénanes ou d'agar comme actif amincissant.
EP 1402874 décrit des compositions acides contenant des polysaccharides sulfatés naturels ou semi-synthétiques comme agents de pénétration ou pour favoriser la pousse des cheveux, pour blanchir la peau, accélérer la teinture des cheveux ou pour un effet tenseur immédiat de la peau
JP 05051311 décrit un mélange de plusieurs types de carraghénane et de gomme de caroube comme agent humectant de la peau.
Cependant, à la connaissance des inventeurs, ces composés n'ont jamais été proposés pour améliorer de façon durable les propriétés de surface de la peau et pour favoriser la maturation de l'enveloppe cornée.

Le présent texte décrit une composition qui contient, dans un milieu physiologiquement acceptable, l'association d'au moins un carraghénane et d'au moins un C-glycoside de formule générale (I) suivante

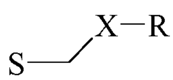

(I)

dans laquelle :

- R représente un radical alkyle, saturé en $C_1$ à $C_{10}$, en particulier en $C_1$ à $C_4$, et pouvant être éventuellement substituée par au moins un radical choisi parmi OH, COOH ou COOR"$_2$, avec R"$_2$ étant un radical alkyle, en $C_1$-$C_4$ saturé,
- S représente un monosaccharide ou un polysaccharide comportant jusqu'à 20 unités sucre, en particulier jusqu'à 6 unités sucre, sous forme pyranose et/ou furanose et de série L et/ou D, ledit mono- ou polysaccharide pouvant être substitué par un groupement hydroxyle obligatoirement libre, et éventuellement une ou plusieurs fonction(s) amine(s) éventuellement protégée(s), et
- X représente un radical choisi parmi les groupements -CO-, -CH(OH)-, -CH ($NH_2$)-, -CH($NHCH_2CH_2CH_2OH$)-, -CH(NHPh)- et -CH($CH_3$)- et en particulier un radical -CO-, -CH(OH)- ou -CH($NH_2$)- et plus particulièrement un radical -CH(OH)-,
- la liaison S-$CH_2$-X représente une liaison de nature C-anomérique, qui peut être $\alpha$ ou $\beta$,

ainsi que leurs sels cosmétiquement acceptables, leurs solvates tels que les hydrates et leurs isomères optiques et géométriques.

La présente invention a pour objet une composition qui contient dans un milieu physiologiquement acceptable l'association d'au moins un carraghénane de forme lambda et d'au moins un C-glycoside de formule générale (I) suivante

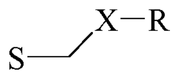

(I)

dans laquelle :

- R représente un radical alkyle linéaire non substitué en $C_1$ à $C_4$,
- S représente un monosaccharide choisi parmi le D-glucose, le D-Xylose, la N-acétyl-D-glucosamine ou le L-fucose, et
- X représente un radical choisi parmi les groupements -CO-, -CH(OH)-, $-CH(NH_2)-$,
- la liaison $S-CH_2-X$ représente une liaison de nature C-anomérique, qui peut être $\alpha$ ou $\beta$,

ainsi que leurs sels cosmétiquement acceptables, leurs solvates tels que les hydrates et leurs isomères optiques et géométriques, avec la concentration en carraghénanes qui est inférieure ou égale à 0,1% par rapport au poids total de la composition.

L'invention a également pour objet une association synergique d'au moins un dérivé C-glycoside tel que défini plus haut et d'au moins un carraghénane de forme lambda, ladite association stimulant de façon synergique la maturation de l'enveloppe cornée, avec la concentration en carraghénanes qui est inférieure ou égale à 0,1% par rapport au poids total de la composition.

L'invention concerne encore l'utilisation d'une telle association ou d'une composition comprenant au moins un C-glycoside et au moins un carraghénane de forme lambda, avec la concentration en carraghénanes qui est inférieure ou égale à 0,1% par rapport au poids total de la composition, pour améliorer, avantageusement de façon rémanente, l'état de surface de la peau en diminuant sa rugosité et/ou en augmentant la transparence ou l'éclat du teint, et pour augmenter l'élasticité de la peau, cet effet étant de préférence rémanent.

Un autre objet de l'invention est un procédé de traitement cosmétique pour améliorer la fonction barrière de la peau, ou pour augmenter l'élasticité et/ou la douceur de la peau, ou pour améliorer la transparence et /ou l'éclat du teint, comprenant l'application sur la peau d'un sujet d'au moins une composition ou une association selon l'invention.

Il a maintenant été mis en évidence que l'association de carraghénanes de forme lambda et de C-glycosides, avec la concentration en carraghénanes qui est inférieure ou égale à 0,1% par rapport au poids total de la composition, a un effet synergique pour favoriser la maturation de l'enveloppe cornée et plus globalement améliorer le processus de différenciation conduisant à la formation du stratum corneum. Les associations ou compositions les contenant permettent ainsi d'améliorer les propriétés biophysiques de la peau, en particulier de l'épiderme et/ou de lutter contre leur diminution. Les propriétés améliorées se traduisent notamment par une amélioration de la fonction barrière de la peau, mais aussi une diminution de la rugosité et/ou une augmentation de la douceur de la surface de la peau.

Il a été montré, dans le cadre de l'invention, que des cornéocytes ayant une bonne maturation, qui se traduit par une enveloppe cornée ayant une enveloppe lipidique constituée, permettant une coloration mesurable au Rouge Nil, ont une rugosité inférieure à celle de cornéocytes immatures, de l'ordre d'un facteur 2 : rugosité de l'ordre de 42 nm pour les cornéocytes matures et de l'ordre de 72nm pour les cornéocytes immatures. Ces cornéocytes immatures présentent un enveloppe fragile et rigide, résultant d'une enveloppe protéique insuffisamment réticulée et d'une enveloppe lipidique non constituée (et donc non identifiable par coloration au Rouge Nil, mais identifiable par une immunohistochimie spécifique de l'involucrine). La rugosité de ces cornéocytes matures et immatures peut notamment être mise en évidence par microscopie atomique (AFM: "atomic force microscopy") et analyse topographique d'image.

En outre, les cornéocytes ayant une maturation complète de l'enveloppe cornée, ont une épaisseur plus faible que celle de cornéocytes immatures.

Ces propriétés des cornéocytes ont une influence importante sur l'aspect visuel de la peau: la transmission et la réflection de la lumière par la peau seront meilleures lorsque la rugosité et l'épaisseur des cornéocytes de surface est diminuée, conduisant à une peau avec un éclat et une transparence augmentée.

En outre, il a été montré de façon surprenante que des cornéocytes présentant une enveloppe cornée mature ont une dureté supérieure à celle des cornéocytes immatures. Le paramètre de dissipation des cornéocytes matures est aussi inférieur à celui des cornéocytes immatures. Cette propriété aura un impact sur le toucher de la peau: plus l'élasticité mécanique et la dureté du support sont élevées, plus la dissipation est faible, et plus le toucher est glissant et doux.

La souplesse de la peau au toucher est aussi améliorée.

Ces propriétés seront conservées même dans des conditions hygrométriques très basses.

Le maintien des propriétés mécaniques face à des conditions extrêmes est le garant du maintien des propriétés physiques de la peau dans des conditions d'extrême sécheresse ou des variations fortes des conditions extérieures.

Les associations et compositions selon l'invention permettent d'améliorer la dureté et l'élasticité des cornéocytes, et de diminuer leur épaisseur et leur rugosité. La mise en oeuvre de l'invention favorise une maturation de bonne

qualité des cornéocytes. L'invention permet ainsi de conférer des propriétés bénéfiques à la peau, de façon durable en particulier

- fonction barrière efficace
- effet hydratant
- élasticité, douceur et texture lisse de la peau,
- morphologie de surface avec une rugosité faible, bonne cohésion des tissus et amélioration de l'aspect visuel de la peau et de sa transparence.

Ces propriétés sont apportées de façon durable à la peau, c'est-à-dire qu'elles persistent plusieurs jours et/ou plusieurs semaines, même si la peau n'est plus en contact avec les associations et compositions selon l'invention.

L'invention a encore pour objet un procédé de traitement cosmétique pour améliorer la fonction barrière et/ou l'hydratation et/ou la résistance à la pollution de la peau caractérisé en ce que l'on applique sur ladite peau une quantité efficace d'au moins une association ou une composition telles que définies précédemment.

Il est connu qu'une augmentation de la sécheresse cutanée est souvent observée avec l'âge, toutefois de tels états de sécheresse cutanée peuvent également se manifester chez les sujets jeunes. En effet, l'état de sécheresse cutanée est un état physiologique qui peut être présent chez des sujets jeunes, sans aucune cause pathologique. Cette sécheresse peut être constitutive, comme souvent chez les individus à peau pâle, mince et/ou fragile.

Par ailleurs, de nombreux facteurs extérieurs peuvent entrainer l'assèchement de la peau ou aggraver l'état d'une peau déjà sèche. Parmi ces facteurs, on peut citer les conditions climatiques difficiles, les rayons solaires, l'exposition à certains agents chimiques ou thérapeutiques.

Sur le plan physiologique, la peau sèche est souvent associée à une baisse du taux d'hydratation cutanée et à une altération de la fonction barrière, mesurée par la perte insensible en eau. Sur le plan sensoriel, elle est notamment caractérisée par une sensation de tiraillement et/ou de tension cutanée. Pour des raisons évidentes, ces manifestations sont sources d'inconfort, voire de douleurs.

Or, dans certaines situations, qu'il s'agisse de vieillissement cutané, de vieillissement actinique, de déséquilibre alimentaire, d'agressions externes répétées, physiques ou chimiques (comme les UV, la pollution, le vent, le froid, l'air conditionné) ou de facteurs psychologiques (fatigue, stress), l'épiderme humain peut présenter des modifications qualitatives ou quantitatives de sa composition et/ou de sa synthèse lipidique.

Les compositions, procédés et utilisations selon l'invention, s'avèrent ainsi tout particulièrement efficaces :

- pour traiter les états de sécheresse cutanée, ,
- pour traiter les peaux sèches,
- pour traiter les démangeaisons et/ou tiraillements associés aux peaux sèches,
- pour restaurer physiologiquement un état d'hydratation convenable au *stratum corneum,*
- pour traiter les peaux sèches hypo-séborrhéiques,
- pour prévenir et/ou réduire les rides liées à une sécheresse cutanée,
- pour améliorer le confort des peaux et cuirs chevelus secs
- pour lutter contre l'aspect terne et/ou atone de la peau conséquence de son dessèchement,
- pour traiter les peaux ayant subi un stress exogène desséchant induit par un produit chimique tel qu'une composition de peeling par exemple, ou induit par un peeling par rayonnement ou encore induit mécaniquement notamment par frottement, au rasage par exemple.

En particulier, les associations et compositions seront utiles comme agent pour améliorer l'hydratation de la peau.

Selon un mode de réalisation de l'invention, le procédé ou l'utilisation selon l'invention visera à prévenir et /ou traiter les peaux sèches et/ou diminuer les signes associés à la sécheresse cutanée, qu'elle soit constitutive ou acquise.

Dans un mode de mise en oeuvre, l'invention visera ainsi à renforcer la résistance de la peau aux stress internes et/ou externes.

Le procédé selon l'invention pourra notamment être appliqué à des sujets âgés de plus de 40 ans, et en particulier de plus de 50 ans.

Selon encore un autre aspect, l'invention concerne l'utilisation d'une association de carraghénane de forme lambda avec la concentration en carraghénanes qui est inférieure ou égale à 0,1% par rapport au poids total de la composition et de C-glycoside, ou un procédé les mettant en oeuvre, pour renforcer la résistance de la peau aux agressions extérieures, en particulier à la pollution et aux agents polluants (tels que les particules, l'ozone, les oxydes d'azote, les oxydes de soufre et/ou les métaux lourds comme notamment le cobalt, le cadmium ou le mercure) ou aux radicaux libres, aux variations brusques de température ou d'humidité relative de l'atmosphère, au vent, à l'air conditionné, aux contraintes mécaniques, notamment à l'irritation provoquée par certains tissus rugueux ou des soins d'hygiène trop

agressifs; cette utilisation peut également viser à renforcer la résistance de la peau aux stress internes, notamment aux modifications induites par des stress psychologiques ou la fatigue qui ont un retentissement sur l'état de surface de la peau.

Par milieu physiologiquement acceptable on entend un milieu compatible avec les matières kératiniques, en particulier la peau, les muqueuses, les cheveux, les poils et les ongles. Il s'agit en particulier d'un milieu cosmétiquement ou dermatologiquement acceptable.
Par peau on entend l'ensemble du revêtement cutané, y compris les muqueuses et le cuir chevelu.

Les C-glycosides de formule I utiles pour la mise en oeuvre de l'invention sont en particulier ceux pour lesquels R désigne un radical alkyle linéaire non substitué en $C_1$ à $C_4$, préférentiellement en $C_1$ à $C_2$ et plus préférentiellement un radical méthyle.

Parmi les groupes alkyle convenant à la mise en oeuvre de l'invention, on peut notamment citer les groupes méthyle, éthyle, isopropyle, n-propyle, n-butyle, t-butyle, isobutyle, sec-butyle.

Selon un mode de réalisation de l'invention, on peut utiliser un dérivé C-glycoside répondant à la formule (I) pour lequel S peut représenter un monosaccharide choisi parmi le D-glucose, le D-xylose, la N-acétyl-D-glucosamine ou le L-fucose, et en particulier le D-xylose.
Préférentiellement, on utilise un dérivé C-glycoside de formule (I) pour lesquels :

- R désigne un radical alkyle linéaire non substitué en $C_1$-$C_4$, notamment $C_1$-$C_2$, en particulier méthyle ;
- S représente un monosaccharide comme décrit précédemment et en particulier choisi parmi le D-glucose, le D-xylose, la N-acétyl-D-glucosamine ou le L-fucose, et en particulier le D-xylose ;
- X représente un groupement choisi parmi -CO-, -CH(OH)-, -CH($NH_2$)-, et préférentiellement un groupement -CH(OH)- .

Les sels acceptables des composés décrits dans la présente invention comprennent des sels non toxiques conventionnels desdits composés tels que ceux formés à partir d'acides organiques ou inorganiques. A titre d'exemple, on peut citer les sels d'acides minéraux, tels que l'acide sulfurique, l'acide chlorhydrique, l'acide bromhydrique, l'acide iodhydrique, l'acide phosphorique, l'acide borique. On peut également citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.
Lorsque le composé de formule (I) comporte un groupe acide, la neutralisation du ou des groupes acides peut être effectuée par une base minérale, telle que LiOH, NaOH, KOH, $Ca(OH)_2$, $NH_4OH$, $Mg(OH)_2$ ou $Zn(OH)_2$; ou par une base organique telle qu'une alkylamine primaire, secondaire ou tertiaire, par exemple la triéthylamine ou la butylamine. Cette alkylamine primaire, secondaire ou tertiaire peut comporter un ou plusieurs atomes d'azote et/ou d'oxygène et peut donc comporter par exemple une ou plusieurs fonctions alcool; on peut notamment citer l'amino-2-méthyl-2-propanol, la triéthanolamine, la diméthylamino-2-propanol, le 2-amino-2-(hydroxyméthyl)-1,3-propanediol. On peut encore citer la lysine ou la 3-(diméthylamino)propylamine.

Les solvates acceptables pour les composés décrits dans la présente invention comprennent des solvates conventionnels tels que ceux formés lors de la dernière étape de préparation desdits composés du fait de la présence de solvants. A titre d'exemple, on peut citer les solvates dus à la présence d'eau ou d'alcools linéaires ou ramifiés comme l'éthanol ou l'isopropanol.
Parmi les dérivés C-glycoside de formule (I), utilisés selon l'invention, on considère tout particulièrement :

1. C-β-D-xylopyranoside-n-propane-2-one ;
2. C-α-D-xylopyranoside-n-propane-2-one ;
3. C-β-D-xylopyranoside-2-hydroxy-propane ;
4. C-α-D-xylopyranoside-2-hydroxy-propane ;
5. C-β-D-xylopyranoside-2-amino-propane ;
6. C-α-D-xylopyranoside-2-amino-propane ;
7. 1-(C-β-L-fucopyranoside)-propane-2-one ;
8. 1-(C-α-L-fucopyranoside)-propane-2-one ;
9. 1-(C-β-L-fucopyranoside)-2-hydroxy-propane ;
10. 1-(C-α-L-fucopyranoside)-2-hydroxy-propane ;
11. 1-(C-β-L-fucopyranoside)-2-amino-propane ;
12. 1-(C-α-L-fucopyranoside)-2-amino-propane ;
13. 1-(C-β-D-glucopyranosyl)-2-hydroxy-propane ;
14. 1-(C-α-D-glucopyranosyl)-2-hydroxy-propane ;

15. 1-(C-β-D-glucopyranosyl)-2-amino-propane ;
16. 1-(C-α-D-glucopyranosyl)-2-amino-propane ;
17. 1-(C-β-L-fucofuranosyl)-propane-2-one ;
18. 1-(C-α-L-fucofuranosyl)-propane-2-one ;
19. 3'-(acétamido-C-β-D-glucopyranosyl)-propane-2'-one ;
20. 3'-(acétamido-C-α-D-glucopyranosyl)-propane-2'-one ;
21. 1-(acétamido-C-β-D-glucopyranosyl)-2-hydroxyl-propane ;
22. 1-(acétamido-C-β-D-glucopyranosyl)-2-amino-propane ;

A titre illustratif et non limitatif des dérivés C-glycoside convenant plus particulièrement à l'invention, on peut notamment citer les dérivés suivants :

- le C-β-D-xylopyranoside-n-propane-2-one,
- le C-α-D-xylopyranoside-n-propane-2-one,
- le C-β-D-xylopyranoside-2-hydroxy-propane,
- le C-α-D-xylopyranoside-2-hydroxy-propane,
- la 1-(C-β-L-fucopyranoside)-propane-2-one,
- la 1-(C-α-L-fucopyranoside)-propane-2-one,
- le 1-(C-β-L-fucopyranoside)-2-hydroxy-propane,
- le 1-(C-α-L-fucopyranoside) -2-hydroxy-propane,
- le 1-(C-β-D-glucopyranosyl)-2-hydroxyl-propane,
- le 1-(C-α-D-glucopyranosyl)-2-hydroxyl-propane,
- la 1-(C-β-L-fucofuranosyl)-propane-2-one,
- la 1-(C-α-L-fucofuranosyl)-propane-2-one,
- , leurs isomères et leurs mélanges.

Selon un mode de réalisation, le C-β-D-xylopyranoside-2-hydroxy-propane ou le C-α-D-xylopyranoside-2-hydroxy-propane, et mieux le C-β-D-xylopyranoside-2-hydroxy-propane, peuvent être avantageusement mis en oeuvre pour la préparation d'une composition selon l'invention.
Selon un mode de réalisation particulier, le dérivé C-glycoside peut être le C-β-D-xylopyranoside-2-hydroxy-propane (ou **HYDROXYPROPYL TETRAHYDROPYRANTRIOL**) se présentant sous forme d'une solution à 30 % en poids en matière active dans un mélange eau/propylène glycol (60/40 % en poids).
Bien entendu, selon l'invention, un dérivé C-glycoside répondant à la formule (I) peut être utilisé seul ou en mélange avec d'autres dérivés C-glycosides et en toute proportion.
Un dérivé C-glycoside convenant à l'invention peut notamment être obtenu par la méthode de synthèse décrite dans le document WO 02/051828.

Les Carraghénanes sont des polysaccharides sulfatés qui constituent les parois cellulaires de diverses algues rouges, à partir desquelles on peut les obtenir. Parmi ces algues rouges on peut citer de façon non limitative Kappaphycus alvarezii, Eucheuma denticulatum, Eucheuma spinosum, Chondrus crispus, Betaphycus gelatinum, Gigartina skottsbergii, Gigartina canaliculata, Sarcothalia crispata, Mazzaella laminaroides, Hypnea musciformis, Mastocarpus stellatus et Iridaea cordata.
Ils comportent de longues chaines galactanes, formée par des motifs disaccharidiques. Ces polysaccharides sont composés d'une alternance de (1→3) β-D-galactopyranose (unité G) et de (1→4) α-galactopyranose (unité D) ou 3,6-anhydro-α-galactopyranose (unité AnGal). Chaque motif sucre peut être sulfaté une ou plusieurs fois en position 2, 3, 4 ou 6. On peut aussi trouver des groupements méthyle, acide pyruviques ainsi que d'autres motifs sucre greffés sur les structures de base précédemment décrite. Les carraghénanes ont été initialement subdivisés en sous-familles en fonction de leur solubilité dans le KCl, puis selon le nombre, la position des groupements sulfates et la présence de ponts 3',6'-anhydro sur les résidus galactopyranosyles. Il existe au moins une quinzaine de carraghénanes répertoriés dont la structure dépend de l'algue d'origine et de la méthode d'extraction. Parmi les plus courants on peut citer les carraghénanes ci-dessous :

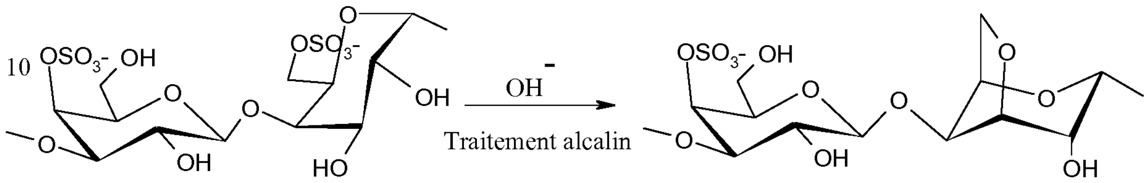


**μ- Carraghénane**
(1→3) β-D-galactopyranose-4-Sulfate-(1→4)-α-D-galactopyranose -6-Sulfate

**κ- Carraghénane**
(1→3) β-D-galactopyranose-4-Sulfate-(1→4) 3,6-anhydro-α-D-galactopyranose

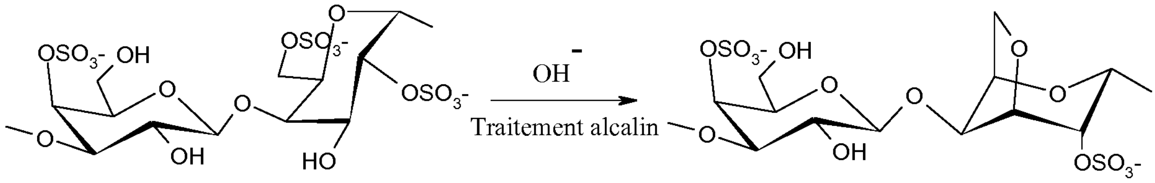


**ν- Carraghénane**
(1→3) β-D-galactopyranose-4-Sulfate-(1→4)-α-D-galactopyranose -2,6-Disulfate

**ι- Carraghénane**
(1→3) β-D-galactopyranose-4-Sulfate-(1→4) 3,6-anhydro-α-D-galactopyranose-2-Sulfate

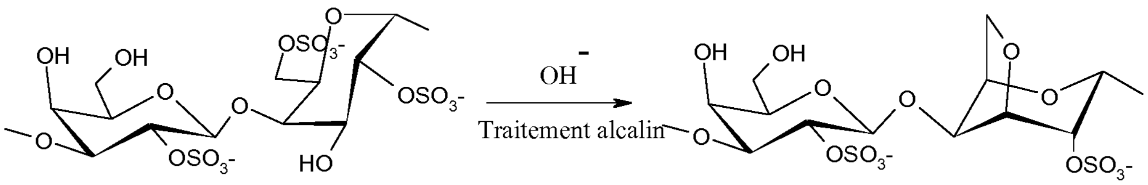


**λ- Carraghénane**
(1→3) β-D-galactopyranose-2-Sulfate-(1→4)-α-D-galactopyranose -2,6-Disulfate

**θ- Carraghénane**
(1→3) β-D-galactopyranose-2-Sulfate-(1→4) 3,6-anhydro-α-D-galactopyranose-2-Sulfate

Ces carraghénanes sont ainsi souvent obtenus sous forme de mélanges de structures différentes telles que, et de façon non limitative, des mélanges de formes κβ, κι, κμ.

Les carraghénanes utilisables pourront notamment être choisis parmi les carraghénanes de type μ, κ, ν, ι, λ θ et leurs mélanges en toutes proportions. Des carraghénanes particulièrement adaptés pour la mise en oeuvre de l'invention sont des carraghénanes de forme lambda. On utilisera notamment des carraghénanes de forme λ, , en particulier des carraghénanes issus de Chondrus Crispus ou Kappaphycus alvarezii.

Les carraghénanes de la présente invention peuvent être utilisés sous forme acide ou sous forme salifiée. A titre de sels acceptables on peut citer, de manière non limitative, les sels de Lithium, Sodium, Potassium, Calcium, Zinc, Ammonium ou les sels obtenus avec une base organique contre ion, telle qu'une alkylamine primaire, secondaire ou tertiaire, notamment la triéthylamine ou la butylamine. Cette alkylamine primaire, secondaire ou tertiaire peut comporter un ou plusieurs atomes d'azote et/ou d'oxygène et peut donc comporter par exemple une ou plusieurs fonctions alcool; on peut notamment citer l'amino-2-méthyl-2-propanol, la triéthanolamine et la diméthylamino-2-propanol, la triéthanolamine. On peut encore citer la lysine ou la 3-(diméthylamino)propylamine. Ces polysaccharides sulfatés peuvent comprendre aussi un mélange de contre-ions parmi ceux définis ci-dessus de façon non limitative.

Ces carraghénanes peuvent aussi faire l'objet d'une sulfatation chimique visant à accroître la proportion de motifs sulfatés ou d'une dé-sulfatation pour diminuer la proportion de ces motifs. Les méthodes de sulfatation des polysaccharides sont connues de l'homme de l'art, mais on pourra se référer de façon non limitative aux travaux de Weiqi Li, Carbohydrate Polymers 605-612, 82(13), 2010. D'une manière générale, la sulfatation est effectuée par réaction d'un agent sulfatant tel que l'acide sulfurique, l'acide chlorosulfonique, les complexes de sulfure de trioxide ($SO_3$) et d'une amine tertiaire (triéthylamine, pyridine) dans un solvant du polysaccharide tel que l'eau, le Diméthylsulfoxide, le Formamide, le Diméthylformamide, la pyridine...Une autre méthode consiste à utiliser une enzyme de type sulfo-transferase qui permet le greffage de groupements sulfates sur les polysaccharides via un donneur de sulfate (généralement le 3'-phosphoadenosine-5'-phosphosulfate (PAPS)).

Le poids moléculaire des polysaccharides utiles pour la présente invention est compris entre 300 et $100.10^6$ Daltons.

Leur poids moléculaire est préférentiellement compris entre $10.10^3$ Da et $10.10^6$ Da. Le taux de soufre des carraghénanes est préférentiellement compris entre 5 et 25% (calculé en poids par rapport au poids total du carraghénane) et plus préférentiellement entre 7 et 20%. Des carraghénanes pourront notamment présenter un taux de soufre d'environ 15% à 20%.

Des carraghénanes particulièrement adaptés pour la mise en oeuvre de l'invention comprennent majoritairement des formes lambda, ou sont sous forme lambda. Par majoritairement on entend que le pourcentage de ce type de chaines dans la composition du produit est supérieure ou égale à 50%, cette proportion pouvant être supérieure ou égale à 80% dans certains modes de réalisation..

De tels carraghénanes peuvent notamment être extraits de Chondrus crispus, tels que ceux commercialisés par la société Cargill, respectivement sous la dénomination Satiagum UTC 30 Carrageenan lamda Cargill et Satiagum UTC 10 Carrageenan lamda Cargill
selon une variante de l'invention, on utilise une association de carraghénane et d'un dérivé aryle-glycoside tel que ceux décrit dans la demande EP 2204162, et notamment un dérivé choisi parmi

- 1-($\alpha,\beta$)-D-xylopyranosyl, 4-(4-hydroxy,3-méthoxy-phenyl) butane-2-ol ;
- 1-($\alpha,\beta$)-D-xylopyranosyl, 4-(4-hydroxy,3-méthoxy-phényl) butane-2-one ;
- 1-($\alpha,\beta$)-D-xylopyranosyl, 4-(4-hydroxyphényl) butane-2-ol ;
- 1-($\alpha,\beta$)-D-xylopyranosyl, 4-phényl butane-2-one ;
- (3E)-1-($\alpha,\beta$)-D-xylopyranosyl, 4-phényl but-3-ène-2-one ;
- (3E)-1-($\alpha,\beta$)-D-xylopyranosyl, 4-[4-hydroxy, (3,5)-diméthoxy-phényl] but-3-ène-2-one ;
- 1-($\alpha,\beta$)-D-xylopyranosyl, 4-[(3,4)-méthylènedioxyphenyl] butane-2-ol ;

Il a été démontré dans le cadre de la présente invention que des associations ou des compositions telles que définies dans ce qui précède sont particulièrement utiles pour améliorer la maturation de l'enveloppe cornée. La mise en oeuvre de l'invention permet ainsi d'améliorer de façon rémanente la fonction barrière de la peau, et donc de prévenir ou diminuer sa dégradation, ou de rétablir son intégrité. L'invention permet de lutter contre les signes associés à un défaut de maturation de l'enveloppe cornée, en les diminuant, en retardant leur survenue et/ou en augmentant la vitesse de leur disparition et le rétablissement des propriétés biophysiques de la peau.

Les quantités des différents actifs seront adaptées par l'homme du métier en fonction de l'effet recherché et du type de formulation utilisé.

Les dérivés C-glycosides seront par exemple utilisés à une concentration comprise entre 0,001 et 10% en poids par rapport au poids total de la composition. On pourra notamment utiliser des concentrations supérieures ou égales à 0,002%, notamment de l'ordre de 0,003%; selon certains modes de réalisation, des concentrations de 0,1 à 3% seront mises en oeuvre.

Une bonne efficacité est obtenue avec des associations et/ou compositions dans lesquelles la concentration en carraghénanes est supérieure ou égale à 0,001% et inférieure à 1% par rapport au poids total de la composition. Avantageusement, la concentration en carraghénanes est inférieure ou égale à 0,1%, notamment inférieure ou égale à 0,06%. On utilisera par exemple des concentrations en carraghénane de 0,001 à 0,03%.

De telles concentrations évitent l'effet tenseur, inconfortable provoqué par l'utilisation de polysaccharides tels que les carraghénanes, et qui n'est pas recherché ici. Elles conduisent à un effet biologique sur la différentiation des cornéocytes,
On obtient une efficacité rémanente des actifs utilisés selon l'invention, sur la transformation de la peau, une amélioration des propriétés biophysiques du stratum corneum lui-même ; le bénéfice perçu n'est pas lié uniquement à l'application de l'actif; en particulier, ce bénéfice persiste même après le rinçage de la peau et l'élimination de la composition ou de l'association selon l'invention.

L'invention pourra en particulier être mise en oeuvre avec des rapports de concentrations en poids C-glycoside/carraghénane supérieur à 25; ce ratio pourra cependant être inférieur ou égal à 3000.

Pour la mise en oeuvre de l'invention l'association et/ ou la composition le contenant, sera appliquée sur la partie de la peau à traiter, en particulier sur le visage, le cou ou les mains, de façon quotidienne ou pluriquotidienne; l'application sera renouvelée tous les jours pendant une période variable selon les effets souhaités, généralement de 3 à 6 semaines, mais pourra être prolongée ou poursuivie en continu.

L'association de carraghénane et de C-glycoside pourra être mise en oeuvre selon l'invention dans une composition pour application topique comprenant en outre au moins un composé choisi parmi les agents hydratants; les agents dépigmentants; les agents anti-glycation; les inhibiteurs de NO-synthase; les agents stimulant la synthèse de macromolécules dermiques ou épidermiques et/ou empêchant leur dégradation; les agents stimulant la prolifération des fibroblastes et/ou des kératinocytes ou stimulant la différenciation des kératinocytes; les agents myorelaxants; les agents tenseurs; les agents anti-pollution et/ou anti-radicalaire, les filtres solaires, et leurs mélanges.

En particulier, l'invention comprend l'application sur la peau d'un actif additionnel diminuant les signes du

vieillissement cutané; un tel actif peut agir en favorisant la prolifération des kératinocytes et/ou des fibroblastes, et peut notamment être choisi parmi les rétinoïdes tel que le rétinol, la vitamine C et ses dérivés, et les monosaccharides.

De façon surprenante, l'association de carraghénane et de C-glycoside telle que définie dans ce qui précède, avec au moins un actif additionnel qui est un monosaccharide choisi parmi le mannose, le rhamnose et leurs mélange présentent une activité améliorée sur l'ensemble des propriétés de la peau, et notamment pour lutter contre la diminution de l'épaisseur de la peau.

Alors que les effets d'une part de stimulation de la différentiation et d'autre part de stimulation de la prolifération sont généralement considérés comme antagonistes, une association de C-glycoside et de carraghénane avec au moins un monosaccharide tel que le rhamnose induit un effet favorable d'augmentation de l'épaisseur de l'épiderme, tout en conservant l'"effet synergique de stimulation de la maturation de l'enveloppe cornée.

Le mannose ou le rhamnose sont capables d'augmenter le nombre de kératinocytes et/ou de fibroblastes, de stimuler le métabolisme des fibroblastes, en stimulant la synthèse des collagènes, en particulier la synthèse de procollagène de type 1; et ainsi de contrer les signes du vieillissement cutané, et en particulier l'atrophie épidermique et/ou dermique liée au vieillissement.

Le mannose est un hexose épimère en C2 du glucose. Le rhamnose (ou 6 deoxy mannose) constitue formellement le produit de désoxygénation du mannose en C6. Les monosaccharides selon l'invention sont sous la forme D ou L du mannose et/ou du rhamnose ou leur mélange, chaque forme pouvant elle-même être l'anomère alpha et/ou béta. Les formes préférées selon l'invention sont le D-mannose ou le L-rhamnose.

Le D-mannose est présent dans les végétaux en particulier certains fruits dont les airelles (canneberges), ou le bois dur (hêtre, bouleau). Le rhamnose est trouvé dans la nature sous forme L. Le D-mannose, ainsi que le L-rhamnose, sont commercialisés, par exemple, par la société Danisco Sweeteners® ou bien la société Symrise.

Selon un mode de réalisation de l'invention, le rapport de concentrations monosaccharide/carraghénane, notamment [rhamnose] / [carraghénane] est supérieur à 1, en particulier supérieur ou égal à 10; dans certains modes de réalisation, ce ratio pourra être supérieur à 50. La concentration en monosaccharide, notamment en rhamnose sera généralement supérieure à 0.1% et de préférence supérieure à 1% en poids, par rapport au poids total de la composition; on utilisera par exemple des concentrations de rhamnose de 0,1 à 5%.

L'invention donc également pour objet une association comprenant au moins un carraghénane, au moins un dérivé C-Glycoside et au moins un monosaccharide tels que définis dans ce qui précède, ou une composition la contenant. En particulier, le dérivé C-glycoside sera le C-β-D-xylopyranoside-2-hydroxy-propane. De préférence, au moins un monosaccharide sera le rhamnose.

Ces associations et compositions seront bien entendu adaptées pour la mise en oeuvre des procédés et utilisation selon l'invention.

L'association triple carraghénane + C-glycoside + monosaccharide permet à la fois, d'avoir

- un effet de surface sur la qualité du stratum corneum, effet sur la maturation et la différentiation de ce stratum, l'amélioration rémanente de la fonction barrière, de la rugosité, de la texture et de l'éclat de la peau ;
- un effet anti-âge sur l'augmentation de l'épaisseur de l'épiderme vivant, ce qui aura pour impact une réduction des ridules et rides.

L'invention a en particulier pour objet un procédé de traitement cosmétique pour diminuer les signes du vieillissement cutané, dans lequel on applique à un individu, une association comprenant comme agent actif au moins un carraghénane, au moins du C-β-D-xylopyranoside-2-hydroxy-propane et au moins du rhamnose.

L'invention sera particulièrement adaptée pour des individus, en particulier des femmes, agés d'au moins 40 ans, et notamment d'au moins 50 ans.

Les signes du vieillissement pourront notamment être choisis parmi les rides et les ridules, la perte d'éclat du teint, l'amincissement de la peau, l'aspect flasque et la perte d'élasticité de la peau.

Par agent actif ou ingrédient actif, on entend plus spécifiquement selon l'invention, un composé qui, lorsqu'il est administré à un sujet, en particulier un sujet humain, joue un rôle biologique direct sur l'organisme, en particulier sur la peau ou ses phanères, en particulier sans améliorer l'effet biologique ou mécanique d'un autre composé présent dans la composition selon l'invention.

La composition selon l'invention comprend au moins une association telle que définie précédemment en association avec un milieu physiologiquement acceptable, en particulier un milieu cosmétiquement ou pharmaceutiquement acceptable.

D'une manière générale, ce milieu peut être anhydre ou aqueux. Il peut ainsi comprendre une phase aqueuse et/ou une phase grasse.

Le milieu physiologiquement acceptable dans lequel les composés selon l'invention peuvent être employés, ainsi que ses constituants, leur quantité, la forme galénique de la composition, son mode de préparation, et son mode d'administration peuvent être choisis par l'homme du métier sur la base de ses connaissances générales en fonction du type de

composition recherchée.

Lorsque la composition est une composition destinée à une administration topique, elle peut se présenter avantageusement sous la forme de solutions aqueuses, hydroalcooliques, d'émulsions huile-dans-eau (H/E) ou eau-dans huile (E/H) ou multiple (triple : E/H/E ou H/E/H), des nanoémulsions, en particulier des nanoémulsions H/E, dont la taille des gouttes est inférieure à 100nm, de gels aqueux, ou de dispersions d'une phase grasse dans une phase aqueuse à l'aide de sphérules, ces sphérules pouvant être des nanoparticules polymériques telles que les nanosphères et les nanocapsules ou des vésicules lipidiques de type ionique et/ou non ionique (liposomes, niosomes, oléosomes). Ces compositions sont préparées selon les méthodes usuelles.

En outre, les compositions utilisables selon l'invention peuvent être plus ou moins fluides et avoir l'aspect d'une crème blanche ou colorée, d'une pommade, d'un lait, d'une lotion, d'un sérum, d'une pâte ou d'une mousse. Elles peuvent être éventuellement appliquées sur la peau sous forme d'aérosol. Elles peuvent aussi se présenter sous forme solide, et par exemple sous forme de stick.

Pour une application locale sur les cheveux ou le cuir chevelu, la composition peut être sous forme de solutions aqueuses, alcooliques ou hydroalcooliques; sous forme de crèmes, de gels, d'émulsions, de mousses; sous forme de compositions pour aérosol comprenant également un agent propulseur sous pression.

Lorsque la composition se présente sous forme aqueuse, notamment sous forme de dispersion, d'émulsion ou de solution aqueuse, elle peut comprendre une phase aqueuse, qui peut comprendre de l'eau, une eau florale et/ou une eau minérale.

Lorsque la composition est une émulsion, la proportion de la phase grasse peut varier d'environ 5 % à 80 % en poids, et de préférence d'environ 2 % à 50 % en poids par rapport au poids total de la composition. Les huiles, les cires, les émulsionnants et les co-émulsionnants utilisés dans la composition sous forme d'émulsion sont choisis parmi ceux classiquement utilisés dans le domaine cosmétique. L'émulsionnant et le co-émulsionnant sont présents, dans la composition, en une proportion allant de 0,3 % à 30 % en poids, et de préférence de 0,5 % à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

La phase huileuse peut comprendre aussi tout additif habituel liposoluble ou lipodispersible comme indiqué ci-après.

Elle peut notamment comprendre des corps gras tels que des cires, des composés pâteux, des alcools gras, des acides gras. La phase huileuse contient au moins une huile, plus particulièrement au moins une huile cosmétique. On entend par "huile" un corps gras liquide à la température ambiante (25°C).

Comme huiles utilisables dans la composition de l'invention, on peut citer par exemple

- les huiles hydrocarbonées d'origine animale, telles que le perhydrosqualène;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple, les huiles de tournesol, de maïs, de soja, de courge, de pépins de raisin, de sésame, de noisette, d'abricot, de macadamia, d'arara, de coriandre, de ricin, d'avocat, les triglycérides des acides caprylique/caprique comme ceux commercialisés par la société Stearineries Dubois ou ceux commercialisés sous les dénominations Miglyol 810, 812 et 818 par la société Dynamit Nobel, l'huile de jojoba, l'huile de beurre de karité, le caprylyl glycol ;
- les esters et les éthers de synthèse, notamment d'acides gras, comme les huiles de formules R1 COOR2 et R1OR2 dans laquelle R1 représente le reste d'un acide gras ou d'unalcool gras comportant de 8 à 29 atomes de carbone, et R2 représente une chaîne hydrocarbonée, ramifiée ou non, contenant de 3 à 30 atomes de carbone, comme par exemple l'huile de Purcellin, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrytyle, l'isopropyl lauroyl sarcosinate, notamment vendu sous le nom commercial Eldew SL 205 de la société Ajinomoto ;
- les hydrocarbures linéaires ou ramifiés, d'origine minérale ou synthétique, tels que les huiles de paraffine, volatiles ou non, et leurs dérivés, la vaseline, les polydécènes, l'isohexadecane, l'isododecane, le polyisobutène hydrogéné tel que l'huile de Parléam, le mélange de n-undécane (C11) et de n-tridécane (C13) commercialisé sous la référence de CETIOL UT par la Société Cognis ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les huiles de silicone volatiles, en particulier cyclopolydiméthylsiloxanes (cyclométhicones) telles que le cyclohexadiméthylsiloxane et le cyclopentadiméthylsiloxane ; lespolydiméthyl-siloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényldiméthicones, les diphénylméthyldiphényl trisiloxanes, les 2-phényléthyltriméthylsiloxysilicates, et les polyméthylphénylsiloxanes ;

- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les résines de silicone telles que la trifluorométhyl-C1-4-alkyldimethicone et la trifluoropropyldimethicone ; et les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries.
Ces corps gras peuvent être choisis de manière variée par l'homme du métier afin de préparer une composition ayant les propriétés, par exemple de consistance ou de texture, souhaitées.

Les émulsions contiennent généralement au moins un émulsionnant choisi parmi les émulsionnants amphotères, anioniques, cationiques ou non ioniques, utilisés seuls ou en mélange, et éventuellement un co-émulsionnant. Les émulsionnants sont choisis de manière appropriée suivant l'émulsion à obtenir (E/H ou H/E). L'émulsionnant et le co-émulsionnant sont généralement présents dans la composition, en une proportion allant de 0,3 à 30 % en poids, et de préférence de 0,5 à 20 % en poids par rapport au poids total de la composition.

Pour les émulsions E/H, on peut citer par exemple comme émulsionnants les dimethicone copolyols tels que le mélange de cyclomethicone et de dimethicone copolyol, vendu sous la dénomination « DC 5225 C » par la société Dow Corning, et les alkyl-dimethicone copolyols tels que le Laurylmethicone copolyol vendu sous la dénomination « Dow Corning 5200 Formulation Aid » par la société Dow Corning et le Cetyl dimethicone copolyol vendu sous la dénomination « Abil EM 90® » par la société Goldschmidt. On peut aussi utiliser comme tensioactif d'émulsions E/H, un organopolysiloxane solide élastomère réticulé comportant au moins un groupement oxyalkyléné, tel que ceux obtenus selon le mode opératoire des exemples 3, 4 et 8 du document US-A-5,412,004 et des exemples du document US-A-5,811,487, notamment le produit de l'exemple 3 (exemple de synthèse) du brevet US-A-5,412,004. et tel que celui commercialisé sous la référence KSG 21 par la société Shin Etsu.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose ; et leurs mélanges tels que le mélange de stéarate de glycéryle et de stéarate de PEG-40.

Ces compositions peuvent être également des émulsions H/E stabilisées par des particules comme par exemple des particules polymériques décrites dans le brevet FR2760641, des polymères amphiphiles réticulés ou non tels que décrits dans les demandes : FR2853543 et FR2819175.

De façon connue, la composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les absorbeurs d'odeurs et les matières colorantes. Les quantités de ces différents adjuvants sont celles classiquement utilisées dans le domaine cosmétique, et par exemple varient d'environ 0,01 % à 10 % du poids total de la composition. Ces adjuvants, selon leur nature, peuvent être introduits dans la phase grasse, dans la phase aqueuse et/ou dans les sphérules lipidiques.

Comme solvants utilisables dans l'invention, on peut citer les alcools inférieurs notamment l'éthanol, l'isopropanol, le dipropylène glycol, le butylène glycol, et le propylène glycol.

Comme gélifiants hydrophiles utilisables dans l'invention, on peut citer à titre d'exemples non limitatifs, les polymères carboxyvinyliques (carbomer®), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium, la silice hydrophobe, l'éthylcellulose et le polyéthylène. Lorsque l'association est administrée par voie orale, la composition la contenant peut être avantageusement sous la forme d'une gélule, d'un comprimé, ou de pilules. Lorsque le monosaccharide est administré par injection cutanée, la composition le contenant peut être en particulier sous la forme d'une solution stérile.

Les compositions de l'invention peuvent contenir d'autres actifs hydrophiles ou lipophiles. Ces actifs additionnels

sont notamment choisis parmi les agents antioxydants, les agents dermo-relaxants ou dermodécontractants, des agents anti-âge, les agents anti-glycation, les agents stimulants la synthèse de macromolécules dermiques ou épidermiques et/ou empêchant leur dégradation, les agents stimulants la prolifération des fibroblastes ou des kératinocytes et/ou la différenciation des kératinocytes, les agents favorisant la maturation de l'enveloppe cornée, les inhibiteurs de NO synthase, les agents stimulant le métabolisme énergétique des cellules et les agents desquamant.

Agents anti-âge :

Parmi les actifs connus pour lutter contre les signes du vieillissement, notamment cutané, on peut citer notamment :
la vitamine B3, le coenzyme Q10 (ou ubiquinone), la vitamine B9, la vitamine E, les dérivés de la vitamine E, tels que le dérivé phosphaté comme par exemple le TPNA® commercialisé par la société Showa Denko, le resvératrol ou ses dérivés, comme par exemple le resveratrate® commercialisé par la société Estée Lauder, le rétinol ou ses dérivés, et leur mélange.

Agents anti-glycation:

Par « agent anti-glycation », on entend un composé prévenant et/ou diminuant la glycation des protéines de la peau, en particulier des protéines du derme, telles que le collagène.
Comme agents anti-glycation, on peut citer notamment les extraits végétaux de la famille des *Ericaceae,* tels qu'un extrait de myrtille (*Vaccinium angusfifollium, Vaccinium myrtillus*), par exemple celui vendu sous la dénomination « BLUEBERRY HERBASOL EXTRACT PG » par la société COSMETOCHEM, l'ergothionéine et ses dérivés, les hydroxystilbènes et leurs dérivés, tels que le resvératrol et le 3,3', 5,5'-tétrahydroxystilbène (ces agents anti-glycation sont décrits dans les demandes FR 2 802 425, FR 2 810 548, FR 2 796 278 et FR 2 802420, respectivement), les dihydroxystilbènes et leurs dérivés, les polypeptides d'arginine et de lysine tels que celui vendu sous la dénomination «AMADORINE®» par la société SOLABIA, le chorhydrate de carcinine (commercialisé par Exsymol sous la dénomination « ALISTIN®»), un extrait d'*Hélianthus annuus* comme l'Antiglyskin® de SILAB, les extraits de vin tel que l'extrait de vin blanc en poudre sur support maltodextrine vendu sous la dénomination « Vin blanc déshydraté 2F » par la société Givaudan, l'acide thioctique (ou acide alpha lipoïque), un mélange d'extrait de busserole et de glycogène marin comme l'Aglycal LS 8777® de Laboratoires Sériobiologiques, un extrait de thé noir comme le Kombuchka® de Sederma et leurs mélanges.
Comme agents anti-glycation préférés, on citera les extraits de myrtille (*Vaccinium myrtillus*) et l'extrait de thé noir.

Agents stimulant la synthèse de macromolécules dermiques et/ou épidermiques et/ou empêchant leur dégradation :

Parmi les actifs stimulant les macromolécules du derme ou empêchant leur dégradation, on peut citer ceux qui agissent :

- soit sur la synthèse du collagène, tels que les extraits de Centella asiatica, les asiaticosides et dérivés ; les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine® ; les peptides de riz tel que le Nutripeptide® de SILAB, le méthylsilanol mannuronate tel que l'Algisium C® commercialisé par Exsymol ; les hormones végétales telles que les auxines et les lignanes ; l'acide folique ; et un extrait de Medicago sativa (alfafa) tel que celui commercialisé par SILAB sous la dénomination Vitanol®; un extrait peptidique de noisette tel que celui commercialisé par la société Solabia sous la dénomination Nuteline C®; et l'arginine.

- soit sur l'inhibition de la dégradation du collagène, en particulier des agents agissant sur l'inhibition des métalloprotéinases (MMP) telles que plus particulièrement les MMP 1, 2, 3, 9 . On peut citer : les rétinoïdes et dérivés, les extraits de medicago sativa tels que le Vitanol® de Silab, un extrait d'aphanizomenon flos-aquae (cyanophycée) commercialisée sous la dénomination Lanablue® par Atrium Biotechnologies, les oligopeptides et les lipopeptides, les lipoaminoacides, l'extrait de malt commercialisé par la société COLETICA sous la dénomination commerciale Collalift® ; les extraits de myrtille ou de romarin ; le lycopène ; les isoflavones, leurs dérivés ou les extraits végétaux en contenant, en particulier les extraits de soja (commercialisé par exemple par la société ICHIMARU PHARCOS sous la dénomination commerciale Flavostérone SB®), de trèfle rouge, de lin, de kakkon; un extrait de litchi ; la DIPALMITOYL HYDROXYPROLINE commercialisé par Seppic sous le nom SEPILIFT DPHP® : Baccharis genistelloide ou Baccharine commercialisé par SILAB, un extrait de moringa tel que l'Arganyl LS 9781® de Cognis ;

l'extrait de sauge décrit dans la demande FR-A-2812544 de la famille des labiées (*salvia officinalis* de la société Flacksmann), l'extrait de Rhododendron, le blueberry extract, un extrait de *vaccinium myrtillus* tel que ceux décrits dans la demande FR-A-2814950.

- soit sur la synthèse de molécules appartenant à la famille des élastines (élastine et fibrilline), tels que : le rétinol et dérivés en particulier le palmitate de rétinol ; l'extrait de *Saccharomyces Cerivisiae* commercialisé par la société LSN sous la dénomination commerciale Cytovitin® ; et l'extrait d'algue *Macrocystis pyrifera* commercialisé par la société SECMA sous la dénomination commerciale Kelpadelie® ; un extrait peptidique de noisette tel que celui commercialisé par la société Solabia sous la dénomination Nuteline C®.

- soit sur l'inhibition de la dégradation de l'élastine tels que l'extrait peptidique de graines de *Pisum sativum* commercialisé par la société LSN sous la dénomination commerciale Parelastyl® ; les héparinoïdes ; et les composés N-acylaminoamides décrits dans la demande WO 01/94381 tels que l'acide {2-[acétyl-(3-trifluorométhyl-phényl)-amino]-3-méthyl-butyrylamino} acétique, autrement nommé N-[N-acétyl, N'-(3-trifluorométhyl)phényl valyl]glycine ou N-acetyl-N-[3-(trifluoromethyl)phenyl]valylglycine ou acetyl trifluoromethyl phenyl valylglycine, ou un ester de celui-ci avec un alcool en $C_1$-$C_6$ ; un extrait de peptides de riz tel que la Colhibin® de Pentapharm, ou un extrait de Phyllanthus emblica tel que l'Emblica® de Rona.

- soit sur la synthèse des glycosaminoglycannes, tels que le produit de fermentation du lait par lactobacillus vulgaris, commercialisé par la société BROOKS sous la dénomination commerciale Biomin yogourth® ; l'extrait d'algue brune Padina pavonica commercialisé par la société ALBAN MÜLLER sous la dénomination commerciale HSP3® ; l'extrait de *Saccharomyces cerevisiae* disponible notamment auprès de la société SILAB sous la dénomination commerciale Firmalift® ou auprès de la société LSN sous la dénomination commerciale Cytovitin®· ; un extrait de *Laminaria ochroleuca* tel que la Laminaïne® de Secma ; l'essence de Mamaku de Lucas Meyer, un extrait de cresson (Odraline® de Silab).

- soit sur la synthèse de la fibronectine, tels que l'extrait de zooplancton Salina commercialisé par la société SEPORGA sous la dénomination commerciale GP4G® ; l'extrait de levure disponible notamment auprès de la société ALBAN MÜLLER sous la dénomination commerciale Drieline® ; et le palmitoyl pentapeptide commercialisé par la société SEDERMA sous la dénomination commerciale Matrixil®.

Parmi les actifs stimulant les macromolécules épidermiques, telles que la fillagrine et les kératines, on peut citer notamment l'extrait de lupin commercialisé par la société SILAB sous la dénomination commerciale Structurine® ; l'extrait de bourgeons de hêtre *Fagus sylvatica* commercialisé par la société GATTEFOSSE sous la dénomination commerciale Gatuline® RC; et l'extrait de zooplancton Salina commercialisé par la société SEPORGA sous la dénomination commerciale GP4G®; Tripeptide de Cuivre de PROCYTE ; un extrait peptidique de *Voandzeia substerranea* tel que celui commercialisé par la société Laboratoires Sérobiologiques sous la dénomination commerciale Filladyn LS 9397®.

De préférence, on utilisera un actif stimulant la synthèse de macromolécules dermiques et/ou épidermiques et/ou empêchant leur dégradation choisi parmi les agents stimulant la synthèse des glycosaminoglycanes, les agents inhibant la dégradation de l'élastine, les agents stimulant la synthèse de la fibronectine, les agents stimulant la synthèse de macromolécules épidermiques, et leurs mélanges.

Encore plus préférentiellement, on utilisera un actif stimulant la synthèse des glycosaminoglycanes choisi parmi un extrait d'algue brune Padina pavonica, un extrait de *Saccharomyces cerevisiae,* un extrait de *Laminaria ochroleuca,* l'essence de Mamaku, un extrait de cresson et leurs mélanges.

Comme actifs préférés stimulant la synthèse de macromolécules dermiques et/ou épidermiques et/ou empêchant leur dégradation, on peut citer :

les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine® ; les peptides de riz tel que le Nutripeptide® de SILAB, le méthylsilanol mannuronate tel que l'Algisium C® commercialisé par Exsymol ; l'acide folique ; un extrait de Medicago sativa (alfafa) tel que celui commercialisé par SILAB sous la dénomination Vitanol®; un extrait peptidique de noisette tel que celui commercialisé par la société Solabia sous la dénomination Nuteline C®; l'arginine ; un extrait d'aphanizomenon flos-aquae (cyanophycée) commercialisée sous la dénomination Lanablue® par Atrium Biotechnologies, l'extrait de malt commercialisé par la société COLETICA sous la dénomination commerciale Collalift® le lycopène ; un extrait de litchi ; un extrait de moringa tel que l'Arganyl LS 9781® de Cognis ; un extrait de *vaccinium myrtillus* tel que ceux décrits dans la demande FR-A-2814950 ; le rétinol et dérivés en particulier le palmitate de rétinol ; l'extrait de *Saccharomyces Cerisiae* commercialisé par la société LSN sous la dénomination commerciale Cytovitin® ; un extrait peptidique de noisette

tel que celui commercialisé par la société Solabia sous la dénomination Nuteline C®; l'acide {2-[acétyl-(3-trifluorométhyl-phényl)-amino]-3-méthyl-butyrylamino} acétique, autrement nommé N-[N-acétyl, N'-(3-trifluorométhyl)phényl valyl]glycine ou N-acetyl-N-[3-(trifluoromethyl)phenyl]valylglycine ou acetyl trifluoromethyl phenyl valylglycine, ou un ester de celui-ci avec un alcool en $C_1$-$C_6$. ; un extrait de peptides de riz tel que la Colhibin® de Pentapharm, ou un extrait de Phyllanthus emblica tel que l'Emblica® de Rona ; l'extrait d'algue brune Padina pavonica commercialisé par la société ALBAN MÜLLER sous la dénomination commerciale HSP3® ; l'extrait de *Saccharomyces cerevisiae* disponible notamment auprès de la société SILAB sous la dénomination commerciale Firmalift® ou auprès de la société LSN sous la dénomination commerciale Cytovitin® ; un extrait de *Laminaria ochroleuca* tel que la Laminaïne® de Secma ; l'essence de Mamaku de Lucas Meyer, l'extrait de lupin commercialisé par la société SILAB sous la dénomination commerciale Structurine® ; l'extrait de bourgeons de hêtre *Fagus sylvatica* commercialisé par la société GATTEFOSSE sous la dénomination commerciale Gatuline® RC.

Agents stimulant la prolifération des fibroblastes ou des kératinocytes et/ou la différenciation des kératinocytes

Les agents stimulant la prolifération des fibroblastes utilisables dans la composition selon l'invention peuvent par exemple être choisis parmi les protéines ou polypeptides végétaux, extraits notamment du soja (par exemple un extrait de soja commercialisé par la société LSN sous la dénomination Eleseryl SH-VEG 8® ou commercialisé par la société SILAB sous la dénomination commerciale Raffermine®) ; un extrait de protéines hydrolysées de soja tel que le RIDULISSE® de SILAB ; et les hormones végétales telles que les giberrellines et les cytokinines ; un extrait peptidique de noisette tel que celui commercialisé par la société Solabia sous la dénomination Nuteline C®.

De préférence on utilisera un agent favorisant la prolifération et/ou la différenciation des kératinocytes.

Les agents stimulant la prolifération des kératinocytes, utilisables dans la composition selon l'invention, comprennent notamment ; le phloroglucinol, l'extrait de feuille d'hydrangea macrophylla comme l'Amacha liquid E® d'Ichimaru Pharcos, un extrait de levure tel que le Stimoderm® de CLR ; l'extrait de *Larrea divaricata* tel que le Capislow® de Sederma, les mélanges d'extraits de papaye, de feuilles d'olivier et de citron tel que la Xyléine® de Vincience, l'extrait de feuille d'hydrangea macrophylla comme l'Amacha liquid E® d'Ichimaru Pharcos, le rétinol et ses esters dont le palmitate de rétinyle, le phloroglucinol, les extraits de tourteaux de noix commercialisés par Gattefosse et les extraits de *solanum tuberosum* tel que le Dermolectine® commercialisé par Sederma.

Parmi les agents stimulant la différenciation des kératinocytes comprennent par exemple les minéraux tels que le calcium; un extrait peptidique de lupin tel que celui commercialisé par la société SILAB sous la dénomination commerciale Structurine®; le beta-sitosteryl sulfate de sodium tel que celui commercialisé par la société SEPORGA sous la dénomination commerciale Phytocohésine® ; et un extrait hydrosoluble de maïs tel que celui commercialisé par la société SOLABIA sous la dénomination commerciale Phytovityl® ; un extrait peptidique de *Voandzeia substerranea* tel que celui commercialisé par la société Laboratoires Sérobiologiques sous la dénomination commerciale Filladyn LS 9397® ; et les lignanes tels que le sécoisolaricirésinol, le rétinol et ses esters dont le palmitate de rétinyl.

Comme actifs stimulant la prolifération des fibroblastes ou des kératinocytes et/ou la différenciation des kératinocytes préférés, on citera des protéines ou polypeptides végétaux, extraits notamment du soja (par exemple un extrait de soja commercialisé par la société LSN sous la dénomination Eleseryl SH-VEG 8® ou commercialisé par la société SILAB sous la dénomination commerciale Raffermine®) ; un extrait de protéines hydrolysées de soja tel que le RIDULISSE® de SILAB ; un extrait peptidique de noisette tel que celui commercialisé par la société Solabia sous la dénomination Nuteline C®; le phloroglucinol, un extrait de levure tel que le Stimoderm® de CLR ; un extrait peptidique de lupin tel que celui commercialisé par la société SILAB sous la dénomination commerciale Structurine®; un extrait hydrosoluble de maïs tel que celui commercialisé par la société SOLABIA sous la dénomination commerciale Phytovityl® ; un extrait peptidique de *Voandzeia substerranea* tel que celui commercialisé par la société Laboratoires Sérobiologiques sous la dénomination commerciale Filladyn LS 9397® ; ou encore le rétinol et ses esters dont le palmitate de rétinyl.

Agents favorisant la maturation de l'enveloppe cornée

On pourra utiliser dans les compositions de l'invention des agents qui interviennent sur la maturation de l'enveloppe cornée qui s'altère avec l'âge et induit une diminution de l'activité des transglutaminases. On peut citer par exemple l'urée et ses dérivés et en particulier l'Hydrovance® de National Starch et les autres actifs mentionnés dans la demande L'OREAL FR2877220.

Inhibiteurs de NO-synthases

L'agent ayant une action d'inhibiteur de NO synthase peut être choisi parmi les OPC (oligomères procyanidoliques) ; les extraits de végétal de l'espèce *Vitis vinifera* notamment commercialisés par la société Euromed sous la dénomination Leucocyanidines de raisins extra, ou encore par la société Indena sous la dénomination Leucoselect®, ou enfin par la société Hansen sous la dénomination Extrait de marc de raisin ; les extraits de végétal de l'espèce *Olea europaea* de préférence obtenus à partir de feuilles d'olivier et notamment commercialisés par la société VINYALS sous forme d'extrait sec, ou par la société Biologia & Technologia sous la dénomination commerciale Eurol® BT ; les extraits d'un végétal de l'espèce *Gingko biloba* de préférence un extrait aqueux sec de ce végétal vendu par la société Beaufour sous le nom commercial Ginkgo biloba extrait standard et leurs mélanges.

Agents stimulant le métabolisme énergétique des cellules

L'actif stimulant le métabolisme énergétique des cellules peut par exemple être choisi parmi la biotine, un extrait de *Saccharomyces cerevisiae* tel que le Phosphovital® de Sederma, le mélange de sels de sodium, de manganèse, de zinc et de magnésium d'acide pyrrolidone carboxylique comme le Physiogenyl® de Solabia, un mélange de gluconate de zinc, de cuivre et de magnésium tel que le Sepitonic M3® de Seppic et leurs mélanges ; un beta-glucan issu de *Saccharomyces cerevisiae* tel que celui commercialisé par la société Mibelle AG Biochemistry ;

Les actifs additionnels pourront en particulier être choisis parmi les agents desquamant et les agents dermo-relaxants.

De manière préférée, l'agent desquamant dans la présente invention est choisi parmi l'acide salicylique, l'acide jasmonique, l'acide gentisique ou un de leurs dérivés, les alpha- ou beta-hydroxyacides, tels que notamment l'acide glycolique, l'acide lactique ou leurs sels. Le composé d'acide salicylique est avantageusement choisi parmi l'acide salicylique et l'acide n-octanoyl-5-salicylique. On utilisera plus particulièrement l'acide n-octanoyl-5- salicylique.

L'agent dermo-relaxant sera en particulier choisi parmi l'adénosine, ses dérivés et analogues. Parmi les analogues d'adénosine utilisables selon l'invention, on citera notamment les agonistes des récepteurs à l'adénosine et les composés augmentant les niveaux intra ou extracellulaires d'adénosine. Des exemples d'analogues d'adénosine comprennent : la 2'-deoxyadénosine ; la 2', 3'-isopropylidene adénosine; la toyocamycine ; la 1-méthyladénosine ; la N-6-methyladénosine ; l'adénosine N-oxyde; le 6-méthylmercaptopurine riboside et le 6-4 chloropurine riboside.. De préférence, on utilisera l'adénosine.

Les exemples qui suivent sont destinés à illustrer l'invention.

Dans ces exemples on se référera aux figures suivantes:

Figure 1: augmentation du taux d'enveloppes cornées matures par rapport au témoin eau
Figure 2:: dureté des cornéocytes matures et immatures en fonction de l'hygrométrie
paramètre de dissipation mécanique tanδ des cornéocytes matures et immatures en fonction de l'hygrométrie

**EXEMPLE 1 : EFFET SYNERGIQUE DE L'APPLICATION DU C-GLYCOSIDE ET DU CARRAGHENANE SUR LA MATURATION DE L'ENVELOPPE CORNEOCYTAIRE**

Les épidermes reconstruits d'Episkin ont été utilisés pour évaluer l'impact de molécules sur la maturation de l'enveloppe cornée.

Les Episkin J7 ont été cultivés avec le carraghénane et les associations carraghénane **+ C-BETA-D-XYLOPYRANOSIDE-2-HYDROXY-PROPANE (HYDROXYPROPYL TETRAHYDROPYRANTRIOL)** pendant 7 jours. Après isolement du stratum, les enveloppes cornées sont extraites.

Trois types d'enveloppes cornées, matures, intermédiaires et immatures sont identifiés et comptées par un logiciel d'analyse d'image développé spécifiquement.

**Matériaux testés :**

Traitement de chaque puits par 30μl de solution véhicule ou actif dans le véhicule Véhicule : Eau ultrapure Biochrom

- Actif 1 : Carraghénane lamba (chondrus crispus - Cargill)- Satiagum UTC 10
- Actif 2 : C-glycoside : C-β-D-xylopyranoside-2-hydroxy-propane (ou 1,5-anhydro-6,8-dideoxy-l-gluco-octitol)
- Mélange : mélange d'actifs comme suit

Tableau des concentrations des actifs

| Produits testés | Nom | CAS n° | Concentration (% p/v) |
|---|---|---|---|
| Véhicule | Eau ultrapure Biochrom | - | - |
| Actif 1 | Satiagum UTC 10 | 9000-07-01 | 0.01% |
| Actif 1 | Satiagum UTC 10 | 9000-07-01 | 0.1% |
| Actif 2 | C-glycoside | 439685-79-7 | 2.5% |
| Association | C-glycoside<br>Satiagum UTC 10 | 439685-79-7<br>9000-07-01 | 2.5%<br>0.01% |
| Association | C-glycoside<br>Satiagum UTC 10 | 439685-79-7<br>9000-07-01 | 2.5%<br>0.1% |

**Traitements :**

Les kits d'Episkin® sont réceptionnés à 7 jours (J7) de culture. La gélose de transport est changée au profit du milieu de différentiation. Chaque échantillon est traité par 30μl de solvant ou d'actif en solution dans le solvant. Les kits ainsi traités sont mis en culture en étuve humide à 37°C sous $CO_2$. Cette procédure (changement du milieu de culture et traitement) est appliquée à J7, J9, J10 et J13.

A J14, les échantillons sont punchés (punch diamètre 4mm). Les punchs sont réservés aux coupes histologiques (coloration **H**ématoxyline **É**osine **S**afran). L'épiderme restant est récupéré et le stratum corneum (sc) est isolé par digestion trypsique (mise en contact 1H à température ambiante solution de trypsine bovine de pancréas à 10% dans tampon Tris-HCI à pH8). Les SC sont rincés dans 3 bains successifs d'eau ultrapure. Puis ils sont séchés pendant 24h en boîte de dessiccation (en présence de gel chaméléon). Les SC sont alors punchés (punch diamètre 4mm) pour la préparation des enveloppes cornées (EC).

Un test de viabilité cellulaire (MTT) est réalisé.

**Extraction des enveloppes cornées :**

Les punchs sont placés dans des microtubes de 2ml et immergés dans 1.5ml de tampon de dénaturation (Tris-HCI 100mM pH 8.5, Dithiotreitol 20mM, SLS 2%, EDTA 5mM). Les échantillons sont portés à 100°C dans un bain sec pendant 10 minutes sans agitation. Ils sont centrifugés (3000 rpm, 10min, 20°C). Les culots sont récupérés. La phase de dénaturation est appliquée 3 fois. Les culots finaux sont repris dans 0.5ml de tampon PBS sans $Ca^{2+}$ et $Mg^{2+}$ et vortexés.

Les suspensions cellulaires sont déposées sur lames fonctionnalisées (SuperFrost Gold Plus à raison de 100μl dans un puits de 15X16mm (GeneFrame), séchés à température ambiante à l'abri de la poussière pendant 24h. Les lames sont fixées au méthanol à -20°C pendant 10min, séchées sous hotte.

<u>Quantification des différents types d'enveloppes cornées (matures, intermédiaires et immatures) : immunomarquage involucrine et contre-coloration lipidique</u> :

Protocole :

Les lames immunomarquées sont visionnées sous microscope (DM6000B Leica) suivant 2 modes d'observation : contraste de phase et fluorescence.

Le contraste de phase fournit une image en niveau de gris nous permettant de visualiser l'ensemble des cellules présentes dans le champ. Le mode fluorescence (jeu de *filtercube*) est utilisé pour visualiser les fluorochromes fixées à l'échantillon :

• Le Nile Red est une sonde fluorescente permettant de localiser les zones hydrophobes plus particulièrement les lipides. Il est mis en évidence par le filtre N2.1.

• L'Alexa Fluor 488 est un fluorochrome sans spécificité particulière. Il est couplé à un anticorps secondaire. Celui-ci reconnaît l'anticorps primaire spécifique de l'involucrine. La visualisation est réalisée grâce au filtre L5.

| Filtercube | Corrélation Fluorochrome/ filtercube | Excitation Range | Excitation Filter | Dichromatic Mirror | Supression Filter |
|---|---|---|---|---|---|
| N2.1 | Nile Red | Green | BP 515-560 | 580 | LP 590 |
| L5 | Alexa Fluor 488 | Blue | BP 480/40 | 505 | BP 527/30 |

- Hydratation des lames dans DPBS 10min à TA
- Saturation dans DBPS / BSA à 5% 20min à TA
- Saturation dans DBPS / BSA à 0.2% 10min à TA
- Dépôt de 100μl AC1 ([Monoclonal anti-involucrine Clone SY5] au 1/100$^{ème}$) 1h à l'obscurité en milieu humide sous agitation légère
  ◦ Rinçages X3 dans DPBS (3 fois 5 min) sous agitation
- Dépôt de 100μl AC2 ([Alexa Fluor® 488 goat anti-mouse IgG (H+L) 2mg/ml - A11001 Invitrogen] au 1/100$^{ème}$) 1h à l'obscurité en milieu humide sous agitation légère
  ◦ Rinçages X3 dans DPBS (3 fois 5 min) sous agitation
- Coloration au Nile Red dans une solution aqueuse à 75% de glycérol pendant 10min à TA
    ◦ Rinçages X3 au DPBS (3 fois 5 min) sous agitation
    ◦ Rinçage rapide à l'acétone
- Montage au citifluor AF1

Les différents types d'enveloppes cornées sont dénombrés à l'aide un logiciel d'analyse d'images (Image J) en utilisant une macro de comptage. On détermine le pourcentage d'augmentation des enveloppes cornées mature par rapport au témoin eau, les résultats sont présentés sur les figure 1 en annexe et dans le tableau ci-dessous.

**Résultats sur l'association C-glycoside + Carraghenane :**

| | Moy | ec |
|---|---|---|
| C-glycoside 2,5% | 22 | 36 |
| Carraghénane 0,01% | 15 | 45 |
| C-glycoside 2,5% + carraghénane 0,01% | 185 | 30 |

Il apparaît une synergie de l'association C-β-D-xylopyranoside-2-hydroxy-propane 2.5% + carraghénane 0.01% sur l'augmentation du taux d'enveloppes cornées matures.
L'augmentation du taux d'enveloppes cornées matures par rapport au placebo (véhicule) est de :

- 185% pour l'association C-β-D-xylopyranoside-2-hydroxy-propane 2.5% + carraghenane 0.01% :
- 22% pour le C-β-D-xylopyranoside-2-hydroxy-propane 2.5%
- 15% pour le carraghenane 0.01 %

**185% > 22% + 15%**

Cette augmentation très significative de la maturation de l'enveloppe cornée aura un impact favorable sur la fonction barrière du stratum corneum, son hydratation et sa résistance face au dessèchement et aux variations d'hygrométrie extérieure.
Dans le cas d'une utilisation de carraghénane pour une concentration supérieure (0,1%), cette synergie n'apparaît pas.

**EXEMPLE 2:** Impact de la maturation sur la rugosité des cornéocytes et le module élastique des cornéocytes = impact sur les propriétés optiques de la peau et le toucher de la peau

Des cornéocytes avec une enveloppe rigide et fragile ont été caractérisés par microscopie atomique de force (AFM). Les cornéocytes sont déposés sur des surfaces de verre. Un scanner piézoélectrique permet un mouvement précis de la cellule latéralement. En appliquant un force faible, la pointe de SiN3 suit la topographie du cornéocyte. Le déplacement vertical du cornéocyte est suivi par la position du faisceau laser au dos de la pointe. L'analyse topographique est réalisée par le logiciel SPIP.
La technique de l'AFM est basée sur la détection des forces d'interaction à courte portée. L'appareil est placé dans une enceinte à hygrométrie et température contrôlées.

Conditions expérimentales d'imagerie :

- pointe ML06E de raideur 0.10 N/m
- images de résolution 512 * 512 pixels
- taille des scans de 45 à 70μm selon la taille des cornéocytes étudiés
- fréquence de 0.5Hz
- set point (ou force d'appui) de 3nN
- température ambiante de 25°C
- hygrométrie variable, selon l'étude

On étudie les paramètres suivants

- Sq: Root Mean Square rugosité
- Smean: hauteur moyenne
- surface projetée
- Volume

$$Sq = \sqrt{\frac{1}{MN}\sum_{k=0}^{M-1}\sum_{l=0}^{N-1}[z(x_k, y_l)]^2}$$

Les images morphologiques sont obtenues avec des informations laterales et verticales.

Résultats:

**Cornéocyte mature** :

Rugosité : Sq=42nm,
Epaisseur (Smean)=81nm

**Cornéocyte immature:**

Rugosité: Sq=72nm,
Epaisseur: (Smean) =159nm

L'épaisseur des cornéocytes matures est significativement plus faible que celle des cornéocytes immature. Le paramètre caractérisant la rugosité est significativement plus faible pour les cornéocytes matures.

**EXEMPLE 3 :** Impact de la maturation sur le module élastique des corneocytes en fonction de l'hygrométrie = impact sur le toucher de la peau et impact sur la rémanence des propriétés physiques même dans des conditions extrêmes (atmosphère très sèche)

Des cornéocytes avec une enveloppe rigide et fragile ont aussi été caractérisés par une technique de nanoindentation à un taux de 70%, en utilisant MTS Nanoindentor XP pour vérifier les propriétés viscoélastiques des cornéocytes:
Par l'application d'une force normale, à l'aide d'un système bobine / aimant et par la mesure du déplacement d'un poinçon indéformable en diamant à l'aide de capteur capacitif, la technique de nanodureté instrumentée permet de déterminer les grandeurs mécaniques comme la dureté et le module d'élasticité du matériau à une profondeur donnée. L'appareil commercial a été spécifiquement adapté à nos besoins (contrôle de la température et du taux d'humidité relative).
Les essais de nano-indentation ont été réalisés à un taux de déformation imposé (P'/P=3.10-2 s-1) et à une charge maximale de 1mN. L'amplitude de la composante sinusoïdale du signal est de 7nm et la fréquence de travail de 32Hz. Un indent est réalisé sur chaque cornéocyte.
L'analyse des résultats est basée sur les valeurs quantitatives des grandeurs mécaniques de dureté, de module élastique et de dissipation visqueuse, entre 10nm et l'enfoncement maximal, soit entre 50nm et 100nm.

Ainsi l'évolution du module d'Young, de la dureté et de tan$\delta$ en fonction de l'enfoncement plastique dans le matériau pourra être mis en évidence et des valeurs de ces grandeurs mécaniques pourront être données.

Remarque : après indentation, il n'est pas observé d'empreinte visible de l'indenteur à la surface du stratum : il n'y a pas de déformation plastique du matériau. La grandeur « dureté » décrite est donc une pression de contact et non la pression d'écoulement plastique du matériau.

Les résultats sont représentés sur la figure 2

Il apparaît :

* dureté
A 75% d'hygrométrie, les cornéocytes matures ont une dureté supérieure à celle des cornéocytes immatures. Le paramètre de dissipation des cornéocytes matures est aussi inférieur à celui des cornéocytes immatures.
Cette différence de propriété mécanique pourrait provenir de la matrice intercellulaire (organisation des filaments de kératine ou différence de composition chimique de la matrice, rôle des NMFs) ou de l'enveloppe. La résistance mécanique de l'enveloppe (en particulier la partie protéique) contribuerait donc à la résistance mécanique globale du cornéocyte.
Cette propriété aura un impact sur le toucher de la peau (plus l'élasticité mécanique et la dureté du support sont élevées, plus la dissipation est faible, et plus le toucher est glissant et doux).
* perméabilité, sensibilité à l'eau
Les propriétés mécaniques des cornéocytes matures évoluent peu en fonction de l'hygrométrie, alors qu'elles sont significativement modifiées pour les cornéocytes immatures (diminution du module, de la dureté et augmentation de la dissipation moléculaire).
Cette modification des propriétés mécaniques pourrait être liée à une plus grande sensibilité à l'eau des cornéocytes immatures. L'hypothèse suivante pourrait être avancée : l'enveloppe lipidique liée hydrophobe et fortement organisée aurait un rôle osmo-protecteur, qui permettrait au cornéocyte de résister aux variations d'hygrométrie.

**EXEMPLE 4: IMPACT DES ASSOCIATIONS TRIPLE CARRAGHENANE + C-GLYCOSIDE + RHAMNOSE SUR L'EPAISSEUR DE L'EPIDERME**

Les épidermes reconstruits d'Episkin ont été utilisés pour évaluer l'impact de molecules sur l'épaisseur de l'épiderme vivant. Les Episkin J7 ont été cultivés avec le carraghenan et les associations du carraghenane avec le C-glycoside (C-$\beta$-D-xylopyranoside-2-hydroxy-propane) et le rhamnose pendant 7 jours.

**MATÉRIAUX TESTÉS :**

- Traitement de chaque puits par 30$\mu$l de solution véhicule ou actif dans le véhicule

    ◦ Véhicule : Eau ultrapure Biochrom
    ◦ Actif 1 : Carraghénane lamba (chondrus crispus - Cargill)- Satiagum UTC 10
    ◦ Actif 2 : 1,5-anhydro-6,8-dideoxy-l-gluco-octitol - Mexoryl SBB

◦ Mélange : mélange d'actifs comme suit

Tableau des concentrations des actifs

| Produits testés | Nom | CAS n° | Concentration (% p/v) |
|---|---|---|---|
| Véhicule | Eau ultrapure Biochrom | - | - |
| Actif 1 | Satiagum UTC 10 | 9000-07-01 | 0.1% |
| Actif 2 | C-glycoside | 439685-79-7 | 2.5% |
| Actif 3 | Rhamnose | 3615-41-6 | 1% |
| Association | C-glycoside<br>Satiagum UTC 10 | 439685-79-7<br>9000-07-01 | 2.5%<br>0.1% |
| Association | C-glycoside<br>Rhamnose | 439685-79-7<br>3615-41-6 | 2.5%<br>1% |
| Association | Rhamnose<br>Satiagum UTC 10 | 3615-41-6<br>9000-07-01 | <br>0.1% |
| Association triple | C-glycoside<br>Satiagum UTC 10<br>Rhamnose | 439685-79-7<br>9000-07-01<br>3615-41-6 | 2.5%<br>0.1%<br>1% |
| Association triple | C-glycoside<br>Satiagum UTC 10<br>Rhamnose | 439685-79-7<br>9000-07-01<br>3615-41-6 | 2.5%<br>0.01%<br>1% |

**TRAITEMENTS :**

Les kits d'Episkin® sont réceptionnés à 7 jours (J7) de culture. La gélose de transport est changée au profit du milieu de différentiation. Chaque échantillon est traité par 30µl de solvant ou d'actif en solution dans le solvant. Les kits ainsi traités sont mis en culture en étuve humide à 37°C sous $CO_2$. Cette procédure (changement du milieu de culture et traitement) est appliquée à J7, J9, J10 et J13.

A J14, les échantillons sont punchés (punch diamètre 4mm). Les punchs sont réservés aux coupes histologiques (coloration **H**ématoxyline **É**osine **S**afran).

Un test de viabilité cellulaire (MTT) est réalisé.

**EFFET DE L'ASSOCIATION TRIPLE SUR L'EPAISSEUR DE L'EPIDERME VIVANT :**

Les coupes histologiques sont colorées suivant la méthode HES (Hématoxyline/Eosine/Safran).

Les coupes, au nombre de 3 par échantillons sont visualisées sous microscope (DM600 LEICA) en lumière blanche, objectif X10. Un cliché est réalisé au centre de chaque coupe. Les mesures de l'épaisseur de l'épiderme vivant s'effectuent à l'aide du logiciel ImageJ préalablement calibré à l'aide du cliché d'un micromètre (correspondance pixel / µm). Une série de 10 mesures est effectuée par cliché.

L'épaisseur de l'épiderme vivant est mesurée de la couche basale à la couche granuleuse.

Il n'apparaît pas d'augmentation significative de l'épaisseur de l'épiderme avec les associations rhamnose 1% + carraghenane 0.1% ou C-glycoside 2.5% + rhamnose 1%.

Il apparaît une augmentation significative de l'épaisseur de l'épiderme (> 10%) pour les associations C-glycoside + carraghenane + rhamnose (augmentation de 10 et 13%).

Il est donc nécessaire d'associer les 3 actifs C-glycoside, carraghenane et rhamnose pour avoir une augmentation de l'épaisseur significative de l'épiderme vivant. Cette augmentation est mesurable pour les 2 taux de carraghenane, 0.01% et 0.1%.

**EFFET DE L'ASSOCIATION TRIPLE SUR L'AUGMENTATION DU TAUX D'ENVELOPPES CORNEES MATURES:**

Le protocole a été décrit dans l'exemple 1. Les résultats sont indiqués ci-dessous.

| | Moy | ec |
|---|---|---|
| C-glycoside 2,5% + carraghénane 0,01% + Rhamnose 1% | 88 | 45 |
| C-glycoside 2,5% + carraghénane 0,1% + rhamnose 1% | 111 | 42 |

Dans le cas de l'association triple « carraghénane + C-glycoside + rhamnose », la mesure du taux des enveloppes cornées matures vivant indique une augmentation significative de ce taux par rapport au témoin « eau ».

L'association triple « carraghénane + C-glycoside + rhamnose » permet donc, à la fois, d'avoir

- un effet de surface sur la qualité du stratum corneum, effet sur la maturation et la différentiation de ce stratum, l'amélioration rémanente de la fonction barrière, de la rugosité, de la texture et de l'éclat de la peau ;
- un effet anti-âge sur l'augmentation de l'épaisseur de l'épiderme vivant, ce qui aura pour impact une réduction des ridules et rides.

Exemple 5: compositions pour application topique

**Compositions pour le soin du visage sous forme de gel aqueux :**

| | Formule 1 (%) |
|---|---|
| Satiagum UTC10 (CARRAGEENAN) | 0.001 |
| HYDROXYPROPYL TETRAHYDROPYRANTRIOL à 30% en matière active dans un mélange eau / propylene glycol 60/40% | 10 |
| RHAMNOSE | 5 |
| SODIUM HYDROXIDE | 0.12 |
| ADENOSINE | 0.1 |
| CARBOMER | 0.4 |
| AMMONIUM POLYACRYLDIMETHYLTAURAMIDE | 0.15 |
| PRESERVATIVE SYSTEM | 0.9 |
| Silicones | 5 |
| ALCOHOL DENAT | 5 |
| BUTYLENE GLYCOL | 7 |
| GLYCERIN | 4 |
| FRAGRANCE | 0.04 |
| AQUA | Qsp 100 |

**Compositions pour le soin du visage sous forme de creme :**

| | Formule 2 (%) |
|---|---|
| Satiagum UTC10 (CARRAGEENAN) | 0.01 |
| HYDROXYPROPYL TETRAHYDROPYRANTRIOL à 30% en matière active dans un mélange eau / propylene glycol 60/40% | 2.5 |
| RHAMNOSE | 1 |
| DISODIUM EDTA | 0.2 |

(suite)

| | Formule 2 (%) |
|---|---|
| CAPRYLOYL SALICYLIC ACID | 0.3 |
| TRIETHANOLAMINE | 1 |
| ISONONYL ISONONANOATE | 7 |
| DIISOPROPYL SEBACATE | 3 |
| XANTHAN GUM | 0.2 |
| SODIUM HYALURONATE | 0.1 |
| CARBOMER | 1 |
| AMMONIUM POLYACRYLDIMETHYLTAURAMIDE | 0.5 |
| DIMETHICONE | 5.5 |
| DIMETHICONE (and) DIMETHICONOL | 2 |
| PEG-12 DIMETHICONE | 0.5 |
| ALCOHOL DENAT. | 3 |
| AQUA | QSP 100 |
| GLYCERIN | 7 |
| PRESERVATIVE SYSTEM | 0.4 |
| ETHYLHEXYL SALICYLATE | 3.5 |
| OCTOCRYLENE | 4.9 |
| BUTYL METHOXYDIBENZOYLMETHANE | 2.1 |
| EMULSYFING SYSTEM | 3,75 |
| PARFUM | 0.4 |
| PRESERVATIVE SYSTEM | 1,1 |
| charge | 3,25 |

## Revendications

**1.** Composition **caractérisée en ce qu'**elle contient dans un milieu physiologiquement acceptable l'association d'au moins un carraghénane de forme lambda et d'au moins un C-glycoside de formule générale (I) suivante

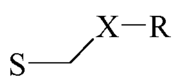

(I)

dans laquelle :

- R représente un radical alkyle linéaire non substitué en $C_1$ à $C_4$,
- S représente un monosaccharide choisi parmi le D-glucose, le D-Xylose, la N-acétyl-D-glucosamine ou le L-fucose, et
- X représente un radical choisi parmi les groupements -CO-, -CH(OH)-, $-CH(NH_2)-$,
- la liaison $S-CH_2-X$ représente une liaison de nature C-anomérique, qui peut être $\alpha$ ou $\beta$,

ainsi que leurs sels cosmétiquement acceptables, leurs solvates tels que les hydrates et leurs isomères optiques et géométriques, avec la concentration en carraghénanes qui est inférieure ou égale à 0,1% par rapport au poids total de la composition.

**2.** Composition selon l'une quelconque des revendications précédentes, **caractérisée en ce qu'**au moins un carraghénanes est extrait d'une algue rouge choisie parmi Chondrus Crispus ou Kappaphycus alvarezii.

**3.** Composition selon l'une quelconque des revendications précédentes, **caractérisé en ce que** le C-glycoside est le C-β-D-xylopyranoside-2-hydroxy-propane.

**4.** Composition selon l'une quelconque des revendications précédentes, **caractérisé en ce que** le rapport des concentrations en poids C-glycoside/ carraghénane est supérieur à 25.

**5.** Association synergique d'au moins un carraghénane de forme lambda tel que défini dans l'une quelconque des revendications 1, 2 et 4 et d'au moins un C-glycoside tel que défini dans l'une quelconque des revendications 1, 3 et 4, **caractérisé en ce que** l'association stimule de façon synergique la maturation de l'enveloppe cornée des cornéocytes, avec la concentration en carraghénanes qui est inférieure ou égale à 0,1% par rapport au poids total de la composition.

**6.** Procédé de traitement cosmétique pour améliorer la fonction barrière de la peau, ou pour augmenter l'élasticité et/ou la douceur de la peau, ou pour améliorer la transparence et /ou l'éclat du teint, **caractérisé en ce qu'**on applique sur la peau d'un sujet au moins une composition selon l'une des revendications 1 à 4 ou une association selon la revendication 5.

**7.** Procédé selon la revendication précédente, **caractérisé en ce qu'**il est destiné à lutter contre la déshydratation cutanée.

**8.** Procédé selon l'une des revendications 6 ou 7, **caractérisé en ce qu'**on applique sur la peau en outre au moins un actif additionnel favorisant la prolifération des kératinocytes et/ou des fibroblastes.

**9.** Procédé selon la revendication précédente, **caractérisé en ce qu'**au moins un actif additionnel est un monosaccharide choisi parmi le mannose, le rhamnose et leurs mélanges.

**10.** Procédé selon la revendication précédente, **caractérisé en ce qu'**on applique au moins une association de C-β-D-xylopyranoside-2-hydroxy-propane, de carraghénane et de rhamnose, ou une composition la contenant.

**11.** Procédé selon l'une quelconque des revendications 9 ou 10, **caractérisé en ce que** le rapport des concentrations monosaccharide/ carraghénane est supérieur à 1.

**12.** Utilisation d'au moins une association synergique de carraghénane de forme lambda et d'au moins un C-glycoside de formule I telle que définie dans la revendication 5, avec la concentration en carraghénanes qui est inférieure ou égale à 0,1% par rapport au poids total de la composition, pour améliorer de façon rémanente l'état de surface de la peau en diminuant sa rugosité et/ou en augmentant la transparence ou l'éclat du teint, et pour augmenter de façon rémanente son élasticité.

**Patentansprüche**

**1.** Zusammensetzung, **dadurch gekennzeichnet, dass** sie in einem physiologisch annehmbaren Medium eine Kombination von mindestens einem Carrageen der lambda-Form und mindestens einem C-Glykosid der folgenden allgemeinen Formel (I)

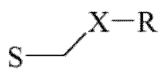

(I)

,

worin:

- R für einen geraden unsubstituierten $C_1$- bis $C_4$-Alkylrest steht,
- S für ein Monosaccharid, ausgewählt aus D-Glucose, D-Xylose, N-Acetyl-D-glucosamin oder L-Fucose, steht und

- X für einen Rest, ausgewählt aus den Gruppen -CO-, -CH(OH)-, $-CH(NH_2)-$, steht,
- die Bindung $S-CH_2-X$ für eine C-Anomer-Bindung, die α oder β sein kann, steht,

sowie deren kosmetisch annehmbare Salze, deren Solvate, wie Hydrate, und deren optische und geometrische Isomere enthält, wobei die Konzentration an Carrageenen kleiner als oder gleich 0,1%, bezogen auf das Gesamtgewicht der Zusammensetzung, ist.

2. Zusammensetzung nach einem der vorhergehenden Ansprüche, **dadurch gekennzeichnet, dass** mindestens ein Carrageen aus einer Rotalge, ausgewählt aus Chondrus crispus oder Kappaphycus alvarezii, extrahiert ist.

3. Zusammensetzung nach einem der vorhergehenden Ansprüche, **dadurch gekennzeichnet, dass** das C-Glykosid C-β-D-Xylopyranosid-2-hydroxypropan ist.

4. Zusammensetzung nach einem der vorhergehenden Ansprüche, **dadurch gekennzeichnet, dass** das Verhältnis der Konzentrationen von C-Glykosid/Carrageen, bezogen auf Gewicht, größer als 25 ist.

5. Synergistische Kombination von mindestens einem Carrageen der lambda-Form nach einem der Ansprüche 1, 2 und 4 und mindestens einem C-Glykosid nach einem der Ansprüche 1, 3 und 4, **dadurch gekennzeichnet, dass** die Kombination auf synergistische Weise die Reifung der verhornten Hülle von Korneozyten stimuliert, wobei die Konzentration an Carrageenen kleiner als oder gleich 0,1%, bezogen auf das Gesamtgewicht der Zusammensetzung, ist.

6. Kosmetisches Behandlungsverfahren zur Verbesserung der Barrierefunktion der Haut oder zur Erhöhung der Elastizität und/oder der Weichheit der Haut oder zur Verbesserung der Transparenz und/oder des Strahlens des Teints, **dadurch gekennzeichnet, dass** man auf die Haut eines Individuums mindestens eine Zusammensetzung nach einem der Ansprüche 1 bis 4 oder eine Kombination nach Anspruch 5 anwendet.

7. Verfahren nach dem vorhergehenden Anspruch, **dadurch gekennzeichnet, dass** es zur Bekämpfung der Hautaustrocknung dient.

8. Verfahren nach einem der Ansprüche 6 oder 7, **dadurch gekennzeichnet, dass** man auf die Haut außerdem mindestens einen zusätzlichen Wirkstoff aufträgt, der die Proliferation von Keratinozyten und/oder Fibroblasten fördert.

9. Verfahren nach dem vorhergehenden Anspruch, **dadurch gekennzeichnet, dass** mindestens ein zusätzlicher Wirkstoff ein aus Mannose, Rhamnose und deren Gemischen ausgewähltes Monosaccharid ist.

10. Verfahren nach dem vorhergehenden Anspruch, **dadurch gekennzeichnet, dass** man mindestens eine Kombination von C-β-D-Xylopyranosid-2-hydroxypropan, Carrageen und Rhamnose oder eine Zusammensetzung, die diese enthält, anwendet.

11. Verfahren nach einem der Ansprüche 9 oder 10, **dadurch gekennzeichnet, dass** das Verhältnis der Konzentrationen von Monosaccharid/Carrageen größer als 1 ist.

12. Verwendung mindestens einer synergistischen Kombination von Carrageen der lambda-Form und mindestens einem C-Glykosid der Formel I nach Anspruch 5, wobei die Konzentration an Carrageenen kleiner als oder gleich 0,1%, bezogen auf das Gesamtgewicht der Zusammensetzung, ist, um auf bleibende Weise den Oberflächenzustand der Haut durch Verringerung ihrer Rauheit und/oder oder durch Erhöhen der Transparenz oder des Strahlens des Teints zu verbessern und auf bleibende Weise ihre Elastizität zu erhöhen.

## Claims

1. Composition **characterized in that** it contains, in a physiologically acceptable medium, the combination of at least one carrageenan of lambda form and of at least one C-glycoside of general formula (I) below

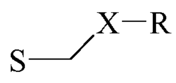

(I)

in which:

- R represents an unsubstituted linear $C_1$-$C_4$ alkyl radical,
- S represents a monosaccharide chosen from D-glucose, D-xylose, N-acetyl-D-glucosamine or L-fucose, and
- X represents a radical chosen from the groups -CO-, -CH(OH)- and $-CH(NH_2)-$,
- the bond $S-CH_2-X$ represents a bond of C-anomeric nature, which may be α or β,

and also the cosmetically acceptable salts thereof, solvates thereof such as hydrates, and optical and geometrical isomers thereof, with the carrageenan concentration which is less than 0.1% relative to the total weight of the composition.

2. The composition according to any one of the preceding claims, **characterized in that** at least one carrageenan is extracted from a red alga chosen from *Chondrus crispus* and *Kappaphycus alvarezii.*

3. Composition according to either one of the preceding claims, **characterized in that** C-glycoside is C-β-D-xylopyranoside-2-hydroxypropane.

4. Composition according to any one of the preceding claims, **characterized in that** the C-glycoside/carrageenan weight concentration ratio is greater than 25.

5. Synergistic combination of at least one carrageenan of lambda form as defined in any one of Claims 1, 2 and 4 and of at least one C-glycoside as defined in any one of Claims 1, 3 and 4, **characterized in that** the combination synergistically stimulates the maturation of the cornified envelope of corneocytes with the carrageenan concentration which is less than 0.1% relative to the total weight of the composition.

6. Cosmetic treatment process for improving the barrier function of the skin, or for increasing the elasticity and/or softness of the skin, or for improving the transparency and/or radiance of the complexion, **characterized in that** at least one composition according to one of claims 1 to 4 or a combination according to claim 5 is applied to the skin of an individual.

7. Process according to the preceding claim, **characterized in that** it is directed toward combating skin dehydration.

8. Process according to either of Claims 6 and 7, **characterized in that** at least one additional active agent which promotes keratinocyte and/or fibroblast proliferation is also applied to the skin.

9. Process according to the preceding claim, **characterized in that** at least one additional active agent is a monosaccharide chosen from mannose, rhamnose, and mixtures thereof.

10. Process according to the preceding claim, **characterized in that** at least one combination of C-β-D-xylopyranoside-2-hydroxypropane, carrageenan and rhamnose, or a composition containing it, is applied.

11. Process according to either of Claims 9 and 10, **characterized in that** the monosaccharide/carrageenan concentration ratio is greater than 1.

12. Use of at least one synergistic combination of carrageenan of lambda form and of at least one C-glycoside of formula I, as defined in Claim 5, with the carrageenan concentration which is less or equal to 0.1% relative to the total weight of the composition, for remanently improving the surface state of the skin by reducing its roughness and/or by increasing the transparency or the radiance of the complexion, and for remanently increasing its elasticity.

Figure 1

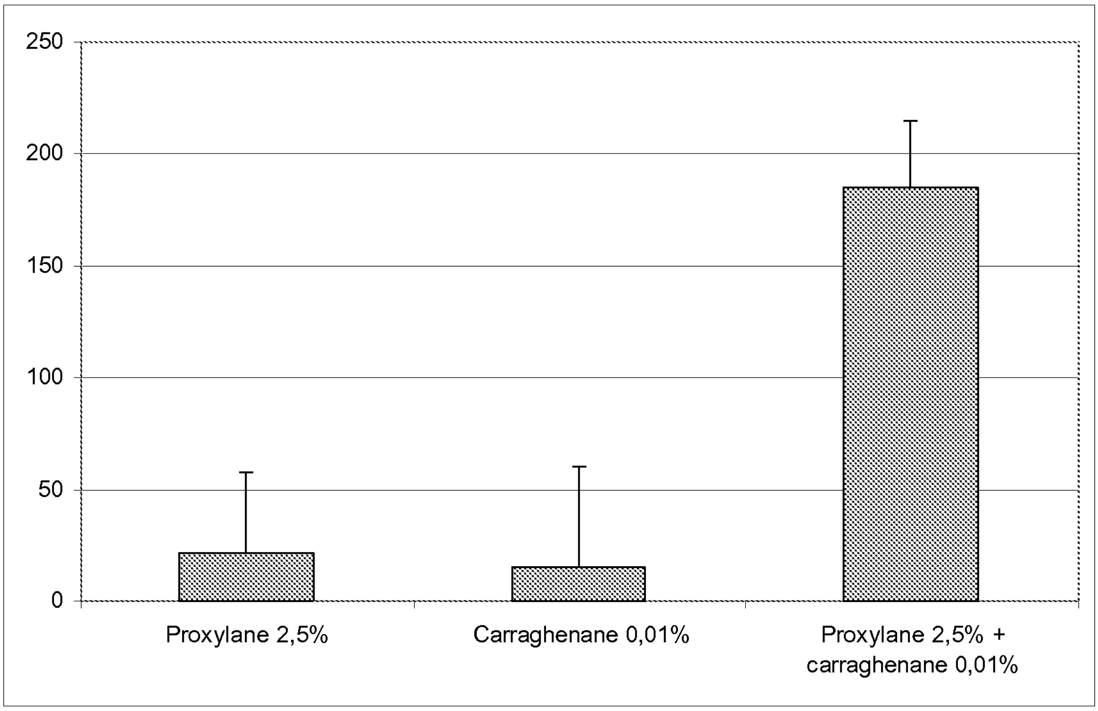
250
200
150
100
50
0
Proxylane 2,5%
Carraghenane 0,01%
Proxylane 2,5% +
carraghenane 0,01%

Figure 2

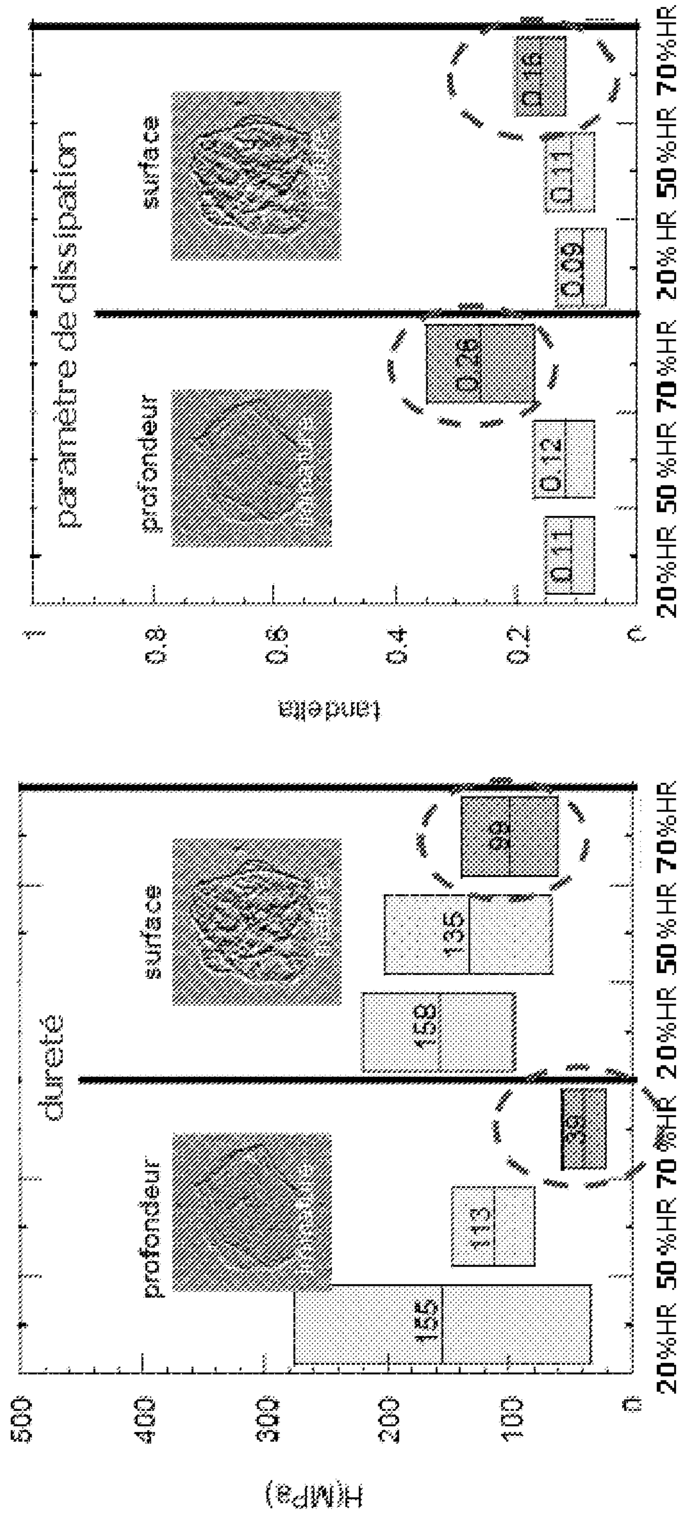
dureté
profondeur
surface
H(MPa)
500
400
300
200
100
0
155
113
39
158
135
99
20%HR 50%HR 70%HR 20%HR 50%HR 70%HR
paramètre de dissipation
profondeur
surface
tandelta
1
0.8
0.6
0.4
0.2
0
0.11
0.12
0.26
0.09
0.11
0.16
20%HR 50%HR 70%HR 20%HR 50%HR 70%HR

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 02051828 A
- FR 2903003
- FR 2917971
- EP 1402874 A
- JP 05051311 B
- EP 2204162 A
- JP 2295912 A
- US 5412004 A
- US 5811487 A
- FR 2760641
- FR 2853543
- FR 2819175
- FR 2802425
- FR 2810548
- FR 2796278
- FR 2802420
- FR 2812544 A
- FR 2814950 A
- WO 0194381 A
- FR 2877220


### Littérature non-brevet citée dans la description


- **HIRAO T et al.** *IFSCC Mag,* 2002, vol. 6 (2), 103-109
- **WEIQI LI.** *Carbohydrate Polymers,* 2010, vol. 82 (13), 605-612